# EUROPEAN PATENT SPECIFICATION

(11) **EP 0 901 482 B1**
(45) Date of publication and mention of the grant of the patent: **02.05.2003**
(21) Application number: 97923193.3
(22) Date of filing: 20.05.1997
(51) Int. Cl.: C07D 307/79, C07D 307/80, C07D 405/12, C07D 405/14, C07D 417/12, C07D 417/14, C07D 417/04, A61K 31/44, A61K 31/445, A61K 31/505, A61K 31/425

(54) **BENZOFURAN CARBOXAMIDES AND THEIR THERAPEUTIC USE**
BENZOFURAN CARBOXAMIDE UND IHRE THERAPEUTISCHE ANWENDUNG
CARBOXAMIDES DE BENZOFURANE ET LEURS UTILISATIONS THERAPEUTIQUES

(30) Priority: 20.05.1996 GB 9610515; 05.12.1996 WO PCT/GB96/03012; 22.04.1997 GB 9708070
(43) Date of publication of application: 17.03.1999
(73) Proprietor: Darwin Discovery Limited, Slough, Berkshire SL1 3WE (GB)
(72) Inventor: DYKE, Hazel Joan, Chiroscience Limited, Cambridge CB4 4WE (GB); LOWE, Christopher, Chiroscience Limited, Cambridge CB4 4WE (GB); MONTANA, John Gary, Chiroscience Limited, Cambridge CB4 4WE (GB); KENDALL, Hannah Jayne, Chiroscience Limited, Cambridge CB4 4WE (GB); SABIN, Verity Margaret, Chiroscience Limited, Cambridge CB4 4WE (GB)
(74) Representative: Perry, Robert Edward
(86) International application number: PCT/GB97/01361
(87) International publication number: WO 97/044337

(56) References cited:
- EP-A- 0 771 794
- WO-A-96/03399
- WO-A-97/20833
- ARZNEIMITTEL FORSCHUNG. DRUG RESEARCH, vol. 21, no. 2, 1971, pages 204-208, XP000602057 AMBROGI V ET AL: "NEW ORAL ANTIDIABETIC DRUGS"

## Description

### Field ofthe Invention

The present invention relates to novel benzofuran carboxamides and thioamides, and to their formulation and use as pharmaceuticals.

### Background of the Invention

EP-A-0637586 discloses benzofuran derivatives, including 4-carboxamides, as acetyleholine esterase inhibitors.

WO-A-9408962 discloses benzofuran analogues as fibrinogen receptor antagonists.

WO-A-9203427 discloses benzofuran-2-carboxamides, with a 3-substituent selected from hydroxy, acyloxy, alkoxy, optionally alkyl-substituted aminoalkoxy, alkylsulphonylamino, optionally alkyl-substituted aminoalkylsulphonyl or arylsulphonylamino, as a remedy for osteoporosis.

EP-A-0685475 discloses benzofuran-2-carboxamides as anti-inflammatory agents.

WO-A-9603399 discloses dihydrobenzofuran-4-carboxamides as inhibitors of phosphodiesterases.

WO-A-9636624 (published 21.11.96; EP-A-0771794) discloses compounds of formula (i) as defined below, of which some may be entitled to a priority date earlier than 20.05.96. In such compounds of earlier date, and with respect to formula (i):
R₁ is optionally-substituted alkoxy;
R₂ and R₃ are each H, optionally-substituted alkyl (e.g. cycloalkyl-substituted alkyl), aryl or heteroaryl, alkanoyl, alkoxycarbonyl or CN; and
R₄ is 4-pyridyl optionally substituted at the 2 and 6 positions by alkyl, alkoxy, COOH, alkanoyl, alkoxycarbonyl, CF₃, NH₂, CN, NO₂ or halogen; and
R₅ is H, alkyl, cycloalkyl, aryl, heteroaryl or aralkyl.

Phosphodiesterases (PDE) and Tumour Necrosis Factor (TNF), their modes of action and the therapeutic utilities of inhibitors thereof, are described in WO-A-9636595, WO-A-9636596 and WO-A-9636611, the contents of which are incorporated herein by reference. The same documents disclose benzofuran derivatives having utility as PDE and TNF inhibitors.

### Summary of the Invention

This invention is based on the discovery of novel compounds that can be used to treat disease states, for example disease states associated with proteins that mediate cellular activity, for example by inhibiting tumour necrosis factor and/or by inhibiting phosphodiesterase IV. According to the invention, compounds of formula (i): 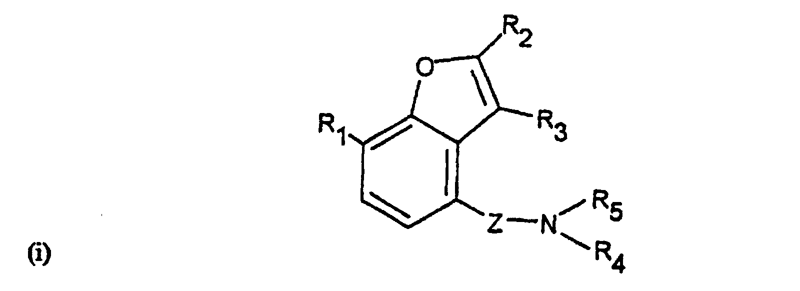 wherein Z is CO or CS;
R₁ represents alkoxy optionally substituted with one or more halogens, OH or thioalkyl;
R₂ and R₃ are the same or different and are each H, R₆, OR₁₀, COR₆, C(=NOR₆)R₆, alkyl-C(=NOR₆)R₆, alkyl-C(=NOH)R₆, C(=NOH)R₆, halogen, NR₈R₉, CF₃, CN, CO₂H, CO₂R₁₀, CONH₂, CONHR₆ or CON(R₆)₂;
R₄ represents H, arylalkyl, heteroarylalkyl, heterocycloalkyl, S(O)ₘR₁₀ or alkyl optionally substituted with one or more substituents chosen from hydroxy, alkoxy, CO₂R₇, SO₂NR₁₁R₁₂, CONR₁₁R₁₂, CN, carbonyl oxygen, NR₈R₉, COR₁₀ and S(O)ₙR₁₀;
R₅ represents aryl, heteroaryl, heterocyclo, arylalkyl, heteroarylalkyl or heterocycloalkyl;
in R₄ and/or R₅, the aryl/heteroaryl/heterocyclo portion is optionally substituted with one or more substituents alkyl-R₁₃ or R₁₃;
R₆ represents R₁₀ optionally substituted at any position with (one or more) R₁₄;
R₇ represents H, alkyl, cycloalkyl, arylalkyl, heteroarylalkyl or heterocycloalkyl;
R₈ represents H, aryl, heteroaryl, heterocyclo, alkyl, cycloalkyl, arylalkyl, heteroarylalkyl, heterocycloalkyl, alkylcarbonyl, alkoxycarbonyl, arylsulphonyl, heteroarylsulphonyl, heterocyclosulphonyl, arylcarbonyl, heteroarylcarbonyl, heterocyclocarbonyl or alkylsulphonyl;
R₁₀ represents alkyl, cycloalkyl, aryl, heteroaryl, heterocyclo, arylalkyl, heteroarylalkyl or heterocycloalkyl;
R₉, R₁₁ and R₁₂ are the same or different, and are each H or R₁₀;
R₁₃ represents alkyl optionally substituted with halogen, alkoxy optionally substituted by halogen, aryl, heteroaryl, heterocyclo, hydroxy, aryloxy, heteroaryloxy, heterocyclooxy, arylalkyloxy, heteroarylalkyloxy, heterocycloalkyloxy, CO₂R₇, CONR₁₁R₁₂, SO₂NR₁₁R₁₂, halogen, -CN, -NR₈R₉, COR₁₀, S(O)ₙR₁₀, or carbonyl oxygen;
R₁₄ represents OH, OR₁₀, carbonyl oxygen, NR₈R₉, CN, CO₂H, CO₂R₁₀, CONR₁₁R₁₂ or COR₁₀;
m represents 1-2; and
n represents 0-2;
and pharmaceutically-acceptable salts;
may be used for the manufacture of a medicament for the treatment of disease states capable of being modulated by inhibition of PDE IV or TNF.

According to another aspect of the invention, novel compounds are those of formula (i) with the proviso that the following compounds are excluded, i.e. those wherein:
R₁ is optionally substituted alkoxy;
R₂ and R₃ are the same or different and are each H, alkyl, cycloalkyl, phenyl, naphthyl or heteroaryl (wherein phenyl, naphthyl or heteroaryl is optionally substituted by 1 to 3 substituents selected from OH, alkoxy, alkanoyl, alkoxycarbonyl, NH₂ and CN), alkanoyl, cycloalkanoyl, alkoxycarbonyl, CN, -(CH₂)₁₋₄-COOR₁₀ when R₁₀ is H, alkyl, cycloalkyl, phenyl, naphthyl, heterocyclo or arylalkyl, or -(CH₂)₁₋₄-CONR₁₁R₁₂ when R₁₁ and R₁₂ are each H, alkyl, cycloalkyl, phenyl, naphthyl, heteroaryl or arylalkyl;
R₄ is H, alkyl or cycloalkyl; and
R₅ is 4-pyridyl optionally substituted at the 3 and/or 5 positions by alkyl, OH, alkoxy, COOH, alkanoyl, alkoxy-CO-, CF₃, NH₂, CN, NO₂ or halogen;

Other novel compounds of the invention are
2-acetyl-7-methoxy-4-*N*-(3,5-dichloropyrid-4-yl)benzofurancarboxamide,
2-acetyl-7-methoxy-4-*N*-(pyrid-4-yl)benzofurancarboxamide,
2-ethyl-7-methoxy-4-*N*-(3,5-dichloropyrid-4-yl)benzofurancarboxamide,
2-acetyl-7-methoxy-4-[*N*-(pyrid-4-yl)-*N*-propyl]benzofurancarboxamide,
2-acetyl-7-methoxy-4-*N*-(3-chloropyrid-4-yl)benzofurancarboxamide,
2-[1-(2,2-dimethylpropyl)]-7-methoxy-4-*N*-(3,5-dichloropyrid-4-yl)benzofurancarboxamide,
*N*-(3,5-dichloropyrid-4-yl)-2-carboxy-7-methoxybenzofurancarboxamide, and
2-ethyl-7-methoxy-4-[*N*-(3-fluoropyrid-4-yl)benzofurancarboxamide, and pharmaceutically-acceptable salts thereof.

Combinations of substituents and/or variables are only permissible if such combinations result in stable compounds.

### Description of the Invention

Suitable pharmaceutically-acceptable salts are pharmaceutically-acceptable base salts and pharmaceutically-acceptable acid addition salts. Certain of the compounds of formula (i) which contain an acidic group form base salts. Suitable pharmaceutically-acceptable base salts include metal salts, such as alkali metal salts for example sodium salts, or organic amine salts such as that provided with ethylenediamine.

Certain of the compounds of formula (i) which contain an amino group form acid addition salts. Suitable acid addition salts include pharmaceutically-acceptable inorganic salts such as the sulphate, nitrate, phosphate, borate, hydrochloride and hydrobromide and pharmaceutically-acceptable organic acid addition salts such as acetate, tartrate, maleate, citrate, succinate, benzoate, ascorbate, methanesulphate, α-ketoglutarate, α-glycerophosphate and glucose-1-phosphate. The pharmaceutically-acceptable salts of the compounds of formula (i) are prepared using conventional procedures.

It will be appreciated by those skilled in the art that some of the compounds of formula (i) may exist in more than one tautomeric form. This invention extends to all tautomeric forms.

It will be appreciated that the compounds according to the invention can contain one or more asymmetrically substituted atoms. The presence of one or more of these asymmetric centers in a compound of formula (i) can give rise to stereoisomers, and in each case the invention is to be understood to extend to all such stereoisomers, including enantiomers, and diastereoisomers and mixtures including racemic mixtures thereof.

When used herein the term alkyl whether used alone or when used as a part of another group includes straight and branched chain alkyl groups containing up to 6 atoms. Alkoxy means an alkyl-O- group in which the alkyl group is as previously described. Aryloxy means an aryl-O- group in which the aryl group is as defined below. Heteroaryloxy means a heteroaryl-O- group and heterocyclooxy means a heterocyclo-O-group in which the heteroaryl and heterocyclo group are as defined below. Arylalkyloxy means an aryl-alkyl-O- group. Heteroarylalkyloxy means a heteroaryl-alkyl-O group and heterocycloalkyloxy means a heterocyclo-alkyl-O- group. Alkylamino means an alkyl-N-group in which the alkyl group is as previously defined, arylamino means aryl-N- and heteroarylamino means an heteroaryl-N- group (aryl and heteroaryl defined below). Thioalkyl means an alkyl-Sgroup. Cycloalkyl includes a non-aromatic cyclic or multicyclic ring system of about 3 to 10 carbon atoms. The cyclic alkyl may optionally be partially unsaturated. Aryl indicates carbocyclic radicals containing about 6 to 10 carbon atoms. Arylalkyl means an aryl-alkylgroup wherein the aryl and alkyl are as described herein. Heteroarylalkyl means a heteroaryl-alkyl group and heterocycloalkyl means a heterocyclo-alkyl group. Alkylcarbonyl means an alkyl-CO- group in which the alkyl group is as previously described. Arylcarbonyl means an aryl-CO- group in which the aryl group is as previously described. Heteroarylcarbonyl means a heteroaryl-CO- group amd heterocyclocarbonyl means a heterocyclo-CO- group. Arylsulphonyl means an aryl-SO₂-group in which the aryl group is as previously described. Heteroarylsulphonyl means a heteroaryl-SO₂- group and heterocyclosulphonyl means a heterocyclo-SO₂- group. Alkoxycarbonyl means an alkyloxy-CO- group in which the alkoxy group is as previously described. Alkylsulphonyl means an alkyl-SO₂- group in which the alkyl group is as previously described. Carbonyl oxygen means a -CO- group. It will be appreciated that a carbonyl oxygen can not be a substituent on an aryl or heteroaryl ring. Carbocyclic ring means about a 5 to about a 10 membered monocyclic or multicyclic ring system which may saturated or partially unsaturated. Heterocyclic ring means about a 5 to about a 10 membered monocyclic or multicyclic ring system (which may be saturated or partially unsaturated) wherein one or more of the atoms in the ring system is an element other than carbon chosen from amongst nitrogen, oxygen or sulphur atoms. Heteroaryl means about a 5 to about a 10 membered aromatic monocyclic or multicyclic hydrocarbon ring system in which one or more of the atoms in the ring system is an element other than carbon, chosen from amongst nitrogen, oxygen or sulphur; if desired, a N atom may be in the form of an N-oxide. Heterocyclo means about a 5 to about a 10 membered saturated or partially saturated monocyclic or multicyclic hydrocarbon ring system in which one or more of the atoms in the ring system is an element other than carbon, chosen from amongst nitrogen, oxygen or sulphur. Halogen means fluorine, chlorine, bromine or iodine.

Compounds of the invention are useful for the treatment of TNF mediated disease states. "TNF mediated disease or disease states" means any and all disease states in which TNF plays a role, either by production of TNF itself, or by TNF causing another cytokine to be released, such as but not limited to IL-1 or IL-6. A disease state in which IL-1, for instance, is a major component, and whose production or action is exacerbated or secreted in response to TNF, would therefore be considered a disease state mediated by TNF. As TNF-β (also known as lymphotoxin) has close structural homology with TNF-α (also known as cachectin), and since each induces similar biologic responses and binds to the same cellular receptor, both TNF-α and TNF-β are considered to be inhibited by compounds of the present invention and thus are herein referred to collectively as "TNF" unless specifically indicated otherwise.

This invention relates to a method for mediating or inhibiting the enzymatic activity or catalytic activity of PDE IV in a mammal in need thereof and for inhibiting the production of TNF in a mammal in need thereof, which comprises administering to said mammal an effective amount of a compound of Formula (i) or a pharmaceutically-acceptable salt thereof.

PDE IV inhibitors are useful in the treatment of a variety of allergic and inflammatory diseases, including: asthma, chronic bronchitis, chronic obstructive airways disease, atopic dermatitis, atopic eczema, urticaria, allergic rhinitis, allergic conjunctivitis, vernal conjunctivitis, inflammation of the eye, allergic responses in the eye, eosinophilic granuloma, psoriasis, Bechet's disease, erythematosis, anaphylactoid purpura nephritis, joint inflammation, arthritis, rheumatoid arthritis and other arthritic conditions such as rheumatoid spondylitis and osteoarthritis, septic shock, sepsis, ulcerative colitis, Crohn's disease, reperfusion injury of the myocardium and brain, chronic glomerulonephritis, endotoxic shock and adult respiratory distress syndrome. In addition, PDE IV inhibitors are useful in the treatment of diabetes insipidus and conditions associated with cerebral metabolic inhibition, such as cerebral senility, senile dementia (Alzheimer's disease), memory impairment associated with Parkinson's disease, depression and multi-infarct dementia. PDE IV inhibitors are also useful in conditions ameliorated by neuroprotectant activity, such as cardiac arrest, stroke and intermittent claudication. PDE IV inhibitors may be useful in the treatment of tardive dyskinesia, ischaemia and Huntingdon's disease.
Additionally, PDE IV inhibitors could have utility as gastroprotectants. A special embodiment of the therapeutic methods of the present invention is the treatment of asthma.

The viruses contemplated for treatment herein are those that produce TNF as a result of infection, or those which are sensitive to inhibition, such as by decreased replication, directly or indirectly, by the TNF inhibitors of Formula (i). Such viruses include, but are not limited to HIV-1, HIV-2 and HIV-3, cytomegalovirus (CMV), influenza, adenovirus and the Herpes group of viruses, such as, but not limited to, *Herpes zoster* and *Herpes simplex.*

This invention more specifically relates to a method of treating a mammal, afflicted with a human immunodeficiency virus (HIV). which comprises administering to such mammal an effective TNF inhibiting amount of a compound of Formula (i) or a pharmaceutically-acceptable salt thereof.

The compounds of this invention may also be used in association with the veterinary treatment of animals, other than humans, in need of inhibition of TNF production. TNF mediated diseases for treatment, therapeutically or prophylactically, in animals include disease states such as those noted above, but in particular viral infections. Examples of such viruses include, but are not limited to feline immunodeficiency virus (FIV) or other retroviral infection such as equine infectious anaemia virus, caprine arthritis virus, visna virus, maedi virus and other lentiviruses.

The compounds of this invention are also useful in treating parasite, yeast and fungal infections, where such yeast and fungi are sensitive to upregulation by TNF or will elicit TNF production *in vivo*. A preferred disease state for treatment is fungal meningitis.

Compounds of the invention may also suppress neurogenic inflammation through elevation of cAMP in sensory neurones. They are, therefore, analgesic, anti-tussive and anti-hyperalgesic in inflammatory diseases associated with irritation and pain.

The compounds of formula (i) are preferably in pharmaceutically-acceptable form. By pharmaceutically-acceptable form is meant, *inter alia*, of a pharmaceutically-acceptable level of purity excluding normal pharmaceutical additives such as diluents and carriers, and including no material considered toxic at normal dosage levels. A pharmaceutically-acceptable level of purity will generally be at least 50% excluding normal pharmaceutical additives, preferably 75%, more preferably 90% and still more preferably 95%.

The invention further provides a process for the preparation of a compound of formula (i), in which R₁ etc, m and n are as defined above. It will be appreciated that functional groups such as amino, hydroxyl or carboxyl groups present in the various compounds described below, and which it is desired to retain, may need to be in protected forms before any reaction is initiated. In such instances, removal of the protecting group may be the final step in a particular reaction sequence. Suitable protecting groups for such functionality will be apparent to those skilled in the art. For specific details, see Protective Groups in Organic Synthesis, Wiley Interscience, TW Greene. Thus the process for preparing compounds of formula (i) in which R₄ contains an -OH comprises deprotecting (for example by hydrogenolysis or hydrolysis) a compound of formula (i) in which R₄ contains an appropriate -OP wherein P represents a suitable protecting group (e.g. benzyl or acetyl).

It will be appreciated that where a particular stereoisomer of formula (i) is required, this may be obtained by conventional resolution techniques such as high performance liquid chromatography or the synthetic processes herein described may be performed using the appropriate homochiral starting material.

A process for the preparation of a compound of formula (i) wherein Z is CO comprises reaction of an appropriate carboxylic acid of formula (ii) with a suitable amine of formula (iii) 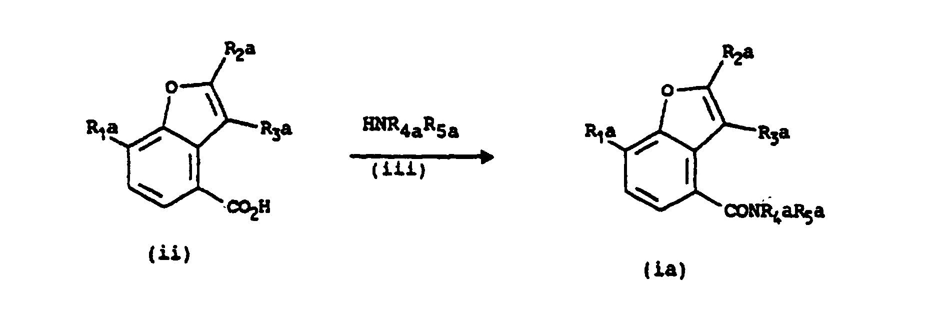 wherein R₁ₐ represents R₁ as defined in relation to formula (i) or a group convertible to R₁ and R₂ₐ-R₅ₐ, similarly represent R₂-R₅ or groups convertible to R₂-R₅ respectively; and thereafter, if required, converting any group R₁ₐ to R₁ and/or R₂ₐ to R₂ and/or R₃ₐ to R₃ and/or R₄ₐ to R₄ and/or R₅ₐ to R₅; and thereafter, if required, converting any group R₁ₐ to R₁ and/or R₂ₐ to R₂ and/or R₃ₐ to R₃ and/or R₄ₐ to R₄ and/or R₅ₐ to R₅. The reaction of a carboxylic acid of formula (ii) with an amine of formula (iii) may be carried out under any suitable conditions known to those skilled in the art. Preferably, the reaction is carried out in the presence of a suitable base, for example an amine such as triethylamine, preferably in an appropriate solvent such as dichloromethane. In some cases a stronger base, such as sodium hydride, and a polar solvent such as dimethylformamide, will be required. Preferably, the carboxylic acid is converted into an acid chloride, mixed anhydride or other activated intermediate prior to reaction with an amine of formula (iii).

Carboxylic acids of formula (ii) and amines of formula (iii) are either commercially available, previously described compounds or are prepared using standard procedures known to those skilled in the art. For example, a carboxylic acid of formula (ii) is conveniently prepared from an appropriate benzofuran of formula (v), using standard procedures known to those skilled in the art. For example, a benzofuran of formula (v) can be formulated to provide an aldehyde of formula (iv), which can then be oxidised to provide the corresponding acid of formula (ii). Alternatively, a benzofuran of formula (v) can be brominated to provide a bromide of formula (vi), which can then be converted into a carboxylic acid of formula (ii), for example by organometal-catalysed carboxylation, such as palladium-catalysed reaction.

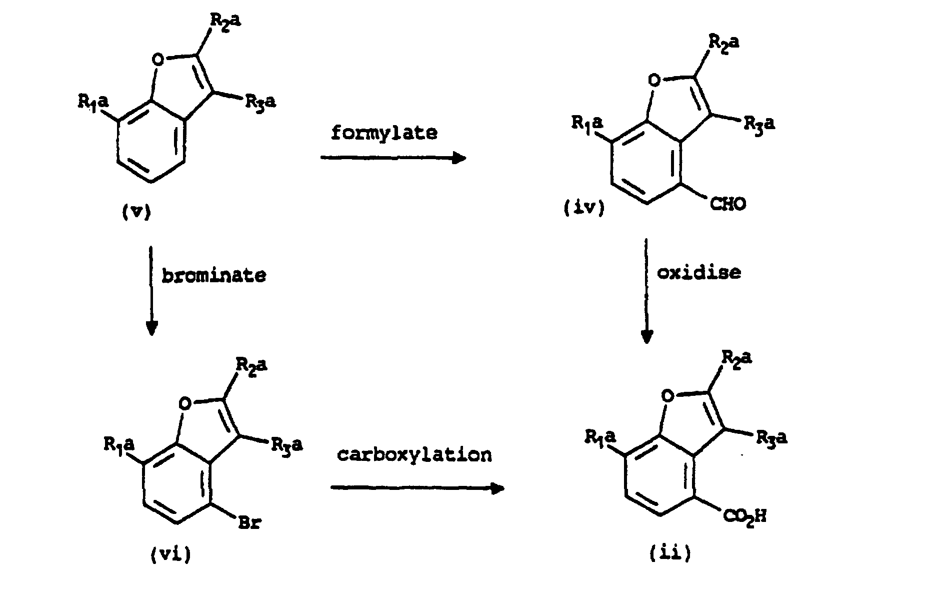

A compound of formula (ia) may also be prepared by reaction of a carboxylic acid of formula (ii) with an amine (iii) to provide a compound of formula (ia) in which R₄ₐ is H, followed by reaction with an agent R₄ₐ Y (vii) in which Y is a suitable leaving group such as a halogen. The first reaction can be carried out as described above. Preferably, the carboxylic acid is converted into an acid chloride, mixed anhydride or other activated intermediate prior to reaction with the amine (iii). The reaction with agent (vii) may be carried out under any suitable conditions known to those skilled in the art. It may be carried out in the presence of a suitable base, e.g. sodium hydride, preferably in an appropriate solvent such as dimethylformamide. Agents (vii) are known or commercially available, or are prepared using standard procedures known to those skilled in the art. Such compounds include alkylating agents such as propyl bromide, acylating agents such as benzoyl chloride and sulphonylating agents such as methanesulphonyl chloride.

Compounds of formula (i) may also be prepared by interconversion of other compounds of formula (i). For example, a compound in which R₄ contains an alkoxy group may be prepared by appropriate alkylation of a compound in which R₄ contains a hydroxy group. Compounds of formula (i) in which Z is CS may be prepared from compounds of formula (i) in which Z is CO using any appropriate conditions known to those skilled in the art, for example by using Lawesson's reagent.

By way of further example, compounds in which R₂ and/or R₃ contain an oxime may be prepared from compounds in which R₂ and/or R₃ contain a carbonyl group. This transformation may be carried out using any appropriate standard conditions known to those skilled in the art. Compounds of formula (i) in which R₂ and/or R₃ contain a carbonyl group may be reduced using standard conditions known to those skilled in the art (for example with sodium borohydride in an appropriate solvent) to provide compounds in which R₂ and/or R₃ contains an alcohol group. Compounds in which R₂ and/or R₃ is alkyl may be prepared by reduction of compounds in which R₂ and/or R₃ is CO-alkyl using standard conditions known to those skilled in the art (for example hydrazine hydrate in the presence of a suitable base in an appropriate solvent). Other transformations may be carried out on compounds of formula (i) in which R₂ and/or R₃ contains a carbonyl group. Such transformations include, but are not limited to, reductive amination and alkylation. Compounds in which R₂ or R₃ contains a CO-alkyl, CO-aryl, CO-heteroaryl, CO-alkylaryl, CO-alkylheterocyclo or CO-alkylheteroaryl group may be prepared from compounds in which R₂ and R₃ contain a CN group by addition of a suitable organometallic reagent (such as a Grignard reagent). Any of the above transformations may be carried out either at the end of the synthesis or on an appropriate intermediate.

A compound of formula (i) or where appropriate a pharmaceutically-acceptable salt thereof and/or a pharmaceutically-acceptable solvate thereof, may be administered *per se* or, preferably, as a pharmaceutical composition also comprising a pharmaceutically-acceptable carrier.

Accordingly, the present invention provides a pharmaceutical composition composing a compound of formula (i) or where appropriate a pharmaceutically-acceptable salt thereof and/or a pharmaceutically-acceptable solvate thereof, and a pharmaceutically-acceptable carrier.

The active compound may be formulated for administration by any suitable route, the preferred route depending upon the disorder for which treatment is required, and is preferably in unit dosage form or in a form that a human patient may administer to himself in a single dosage. Advantageously, the composition is suitable for oral, rectal, topical, parenteral administration or through the respiratory tract. Preparations may be designed to give slow release of the active ingredient.

The term parenteral as used herein includes subcutaneous injections, intravenous, intramuscular, intrasternal injection or infusion techniques. In addition to the treatment of warm-blooded animals such as mice, rats, horses, cattle, sheep, dogs, cats, etc, the compounds of the invention are effective in the treatment of humans.

The compositions of the invention may be in the form of tablets, capsules, sachets, vials, powders, granules, lozenges, suppositories, reconstitutable powders, or liquid preparations such as oral or sterile parenteral solutions or suspensions. Topical formulations are also envisaged where appropriate.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers for example microcrystalline cellulose, lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically-acceptable wetting agents such as sodium lauryl sulphate.

The solid oral compositions may be prepared by conventional methods of blending, filling, tabletting or the like. Repeated blending operations may be used to distribute the active agent throughout those compositions employing large quantities of fillers.

Such operations are of course conventional in the art. The tablets may be coated according to methods well known in normal pharmaceutical practice, in particular with an enteric coating.

Oral liquid preparations may be in the form of, for example, emulsions, syrups or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia, nonaqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

Compositions may also suitably be presented for administration to the respiratory tract as a snuff or an aerosol or solution for a nebuliser, or as a microfine powder for insufflation, alone or in combination with an inert carrier such as lactose. In such a case the particles of active compound suitably have diameters of less than 50 µm, such as from 0.1 to 50 µm, preferably less than 10 µm, for example from 1 to 10 µm, 1 to 5 µm or from 2 to 5 µm. Where appropriate, small amounts of other anti-asthmatics and bronchodilators for example sympathomimetic amines such as isoprenaline, isoetharine, salbutamol, phenylephrine and ephedrine; corticosteroids such as prednisolone and adrenal stimulants such as ACTH may be included.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilised before filling into a suitable vial or ampoule and sealing.

Advantageously, adjuvants such as local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% to 99% by weight, preferably from 10-60% by weight, of the active material, depending on the method of administration.

Compounds of formula (i), or if appropriate a pharmaceutically-acceptable salt thereof and/or a pharmaceutically-acceptable solvate thereof may also be administered as a topical formulation in combination with conventional topical excipients.

Topical formulations may be presented as, for instance, ointments, creams or lotions, impregnated dressings, gels, gel sticks, spray and aerosols, and may contain appropriate conventional additives such as preservatives, solvents to assist drug penetration and emollients in ointments and creams. The formulations may contain compatible conventional carriers, such as cream or ointment bases and ethanol or oleyl alcohol for lotions.

Suitable cream, lotion, gel, stick, ointment, spray or aerosol formulations that may be used for compounds of formula (i) or if appropriate a pharmaceutically-acceptable salt thereof, are conventional formulations well known in the art, for example, as described in standard text books such as Harry's Cosmeticology published by Leonard Hill Books, Remington's Pharmaceutical Sciences, and the British and US Pharmacopoeias.

Suitably, the compound of formula (i), or if appropriate a pharmaceutically-acceptable salt thereof, will comprise from about 0.5 to 20% by weight of the formulation, favourably from about 1 to 10%, for example 2 to 5%.

The dose of the compound used in the treatment of the invention will vary in the usual way with the seriousness of the disorders, the weight of the sufferer, and the relative efficacy of the compound. However, as a general guide suitable unit doses may be 0.1 to 1000mg, such as 0.5 to 200, 0.5 to 100 or 0.5 to 10mg, for example 0.5, 1, 2, 3, 4 or 5mg; and such unit doses may be administered more than once a day, for example 2, 3, 4, 5 or 6 times a day, but preferably 1 or 2 times per day, so that the total daily dosage for a 70kg adult is in the range of about 0.1 to 1000mg, that is in the range of about 0.001 to 20 mg/kg/day, such as 0.007 to 3, 0.007 to 1.4, 0.007 to 0.14 or 0.01 to 0.5mg/kg/day, for example 0.01, 0.02, 0.04, 0.05, 0.06, 0.08, 0.1 or 0.2 mg/kg/day, and such therapy may extend for a number of weeks or months.

When used herein the term "pharmaceutically-acceptable" encompasses materials suitable for both human and veterinary use.

The following Examples illustrate the invention.

### Intermediate 1 2-Acetyl-7-methoxybenzofuran-4-carbonyl chloride

2-Acetyl-7-methoxybenzofuran-4-carboxylic acid (0.12g) was suspended in anhydrous dichloromethane (4ml) at room temperature under nitrogen and oxalyl chloride (0.1ml) added followed by 3 drops of *N,N*-dimethylformamide. Evaporation *in vacuo* after 2 hours afforded the title compound as a yellow solid (∼0.5g).
TLC R_{f} 0.60 (50%ethyl acetate in hexane)

### Intermediate 2 2-Acetyl-7-methoxybenzofuran-4-carboxylic acid

A mixture of 2-acetyl-4-bromo-7-methoxybenzofuran (5g), triphenylphosphine (98mg), bis(triphenylphosphine)palladium(II)chloride (261 mg), triethylamine (2.85ml) and water (1ml) in tetrahyrofuran (25ml) was purged with carbon monoxide gas in a Parr pressure reactor at 110psi. This was heated to 110°C (pressure now 220psi) and left for a week. On cooling and release of pressure the mixture was dissolved in 50% dichloromethane-water (200ml) and taken to pH12 using aqueous sodium hydroxide(1M). The separated aqueous phase was acidified to pH1 using dilute hydrochloric acid(1M) and the resultant slurry extracted with dichloromethane (3x100ml) then ethyl acetate (100ml). These combined organic extracts were dried over magnesium sulphate, filtered and evaporated *in vacuo* to afford a yellow solid (2.58g).
TLC R_{f} 0.61 (ethyl acetate)

### Intermediate 3 2-Acetyl-4-bromo-7-methoxybenzofuran

A solution of bromine (5.5ml) in methanol (100ml) was added dropwise to a suspension of 2-acetyl-7-methoxybenzofuran (20g) in methanol (300ml) at 0°C. The ice bath was removed immediately and the mixture allowed to warm to room temperature. After 1 hour conversion was incomplete, so further bromine (0.75ml) in methanol (25ml) was added and the mixture stirred overnight. The reaction was quenched using aqueous sodium metabisulphite solution (300ml) producing a precipitate that was filtered off and dried *in vacuo* to afford a brown solid (17.4g).
TLC R_{f} 0.90 (ethyl acetate)

### Intermediate 4 2-Ethyl-7-methoxybenzofuran-4-carboxylic acid

2-Methyl-2-butene (9g) was added to a solution of 2-ethyl-7-methoxybenzofurancarboxaldehyde (5g) in 2-methyl-2-propanol (125ml). A solution of sodium dihydrogen phosphate monohydrate (20.7g) in water (15ml) was added, followed by sodium chlorite (11.05g). The resultant heterogeneous mixture was stirred vigorously for 30 minutes and then diluted with water (125ml). The mixture was adjusted to pH 4 by the addition of 2M hydrochloric acid. The mixture was extracted with ethyl acetate (3x200ml) and the combined organic extracts were washed with water (2x200ml). The organic solution was concentrated to about 100ml and then cooled to 10°C. The resultant precipitate was collected by filtration and dried at 50°C *in vacuo* to afford a beige solid (4g).
mp 215-216°C

The following compound was prepared using the above procedure.

### Intermediate 5 2-[1-(2,2-Dimethylpropyl)]-7-methoxybenzofuran-4-carboxylic acid

Prepared from 2-[1-(2,2-dimethylpropyl)]-7-methoxybenzofuran-4-carboxaldehyde (2.14g). The title compound (1.81g) was obtained as a pale yellow solid.
mp 173-174°C

### Intermediate 6 4-Amino-3-chloropyridine

A solution of 4-aminopyridine (4.0g) in concentrated hydrochloric acid (50ml) was treated at 80-85°C with an aqueous solution of hydrogen peroxide (13.5% w/v). The solution was cooled to 0°C. After 30 minutes, the solution was carefully treated with an aqueous sodium hydroxide solution (50%w/v) maintaining the temperature below 15°C. The white solid produced was obtained by filtration and air dried to afford a white solid
(4.9g).
R_{f} 0.36 (ethyl acetate).
mp 65-67°C.

### Intermediate 7 4-N-(Propylamino)pyridine

4-Aminopyridine (0.499g) and propionaldehyde (0.5g) in dichloromethane (50ml) under an inert atmosphere were stirred at ambient temperature for 1.5 hours. Sodium triacetoxyborohydride (2.7g) was added and left overnight. The reaction mixture was washed with aqueous sodium bicarbonate (2x40ml) and extracted into dilute hydrochloric acid (2x40ml). These acidic extracts were basified using potassium hydroxide pellets and extracted into dichloromethane (2x80ml). The combined organic extracts were dried over anhydrous magnesium sulphate, filtered and evaporated *in vacuo* to yield an oily residue (0.11g).
TLC R_{f} 0.49 (10%methanol in ethyl acetate).

### Intermediate 8 2-Ethyl-7-methoxy-4-N-(3-carboethoxyphenyl)benzofuran carboxamide

2-Ethyl-7-methoxybenzofuran-4-carbonyl chloride (1.0g) was added to a solution of ethyl 3-aminobenzoate (0.72g) in dichloromethane (30ml) at room temperature under an inert atmosphere and the reaction mixture stirred at room temperature overnight. The mixture was poured into dilute aqueous hydrochloric acid and extracted with ethyl acetate (2 x 50ml). The combined organic extracts were washed with water (50ml), brine (50ml), dried (magnesium sulphate) and evaporated *in vacuo* to yield the title compound (1.39g) as a white solid.
mp 159-161°C.

The following compound was prepared according to the above procedure:

### Intermediate 9 2-Ethyl-7-methoxy-4-N-(4-carboethoxypheoyl)benzofuran carboxamide

Prepared from 2-ethyl-7-methoxybenzofuran-4-carbonyl chloride (1.3g) and ethyl 4-aminobenzoate (1.0g) to yield the title compound (0.76g) as a white solid.
TLC R_{f} 0.18 (25% ethyl acetate in hexane)

### Intermediate 10 2-[1-(2,2-Dimethylpropyl)]-7-methoxybenzofuran-4-carboxaldehyde

Phosphorus oxychloride (1.64ml) was added dropwise to DMF (1ml) at 0°C under nitrogen and stirred for 10 minutes. A solution of 2-[1-(2,2-dimethylpropyl)]-7-methoxybenzofuran (1.92g) in DMF (3.5ml) was then added. A pale yellow solid formed and the reaction mixture was heated to 100°C for 2h. The reaction mxture was allowed to cool to room temperature overnight. A solution of 50% aqueous sodium acetate trihydrate (20ml) was added cautiously and the resultant mixture was extracted with MTBE (3 x 25ml). The combined organic phases were washed with water (2 x 20ml), saturated aqueous sodium hydrogen carbonate solution (20ml) and brine (30ml). The solution was dried (magnesium sulphate) and concentrated *in vacuo* to provide the title compound (2.14g) as a light brown oil.
TLC R_{f} 0.25 (5% ethyl acetate in hexane)

### Intermediate 11 2-[1-(2,2-Dimethylpropyl)]-7-methoxybenzofuran

Sodium hydroxide (2.89g) was added to a solution of *o*-vanillin (10g) in ethanol (230ml) at 40°C. After 10 minutes, 1-bromopinacolone (9.7ml) was added and the resultant mixture was heated at 60°C for 4h then at reflux for a further 4h. The reaction mixture was cooled to room temperature and then concentrated *in vacuo.* The residue was partitioned between ethyl acetate (100ml) and 0.2% aqueous sodium hydroxide solution (100ml). The aqueous layer was extracted with ethyl acetate ( 2 x 75ml) and the combined organic extracts were washed with water (100ml) and brine (100ml). The solution was dried (magnesium sulphate) and concentrated *in vacuo* to furnish 2-[1-(2,2-dimethyl-1-oxopropyl)]-7-methoxybenzofuran as a brown oil.

Hydrazine hydrate (3.2ml) was added to a stirred suspension of 2-[1-(2,2-dimethyl-1-oxopropyl)]-7-methoxybenzofuran (3.0g) in ethylene glycol (38ml). The reaction mixture was heated to 65°C for 1h, then heated at reflux for 1.75h to afford a yellow solution. After cooling to room temperature, water (50ml) was added and the mixture extracted with dichloromethane (3 x 50ml). The combined organic extracts were washed with 2M aqueous hydrochloric acid (15ml), water (3 x 20ml) and brine 50ml). The solution was dried (magnesium sulphate) and concentrated *in vacuo.* Purification by column chromatography on silica, eluting with 5% ethyl acetate in hexane yielded the title compound (1.92g) as a colourless oil.
TLC R_{f} 0.35 (5% ethyl acetate in hexane)

### Intermediate 12 2-Ethyl-7-methoxybenzofuran-4-carbonyl chloride

2-Ethyl-7-methoxybenzofuran-4-carboxylic acid (4.05g) was heated in dry toluene (100ml) with thionyl chloride (14ml) under nitrogen at 90°C for 2h. The solution was evaporated to dryness *in vacuo* and azeotroped with dry toluene (2 x 50ml) to afford the title compound (4.4g) as an off-white solid.
mp 100-102°C

### Intermediate 13 Methyl 7-methoxybenzofuran-2-carboxylate

7-Methoxybenzofuran-2-carboxylic acid (10g) and methanol (110ml) were combined under nitrogen and cooled to 0°C. Acetyl chloride (11ml) was then added and stirring continued for 18h. Removal of the solvent afforded an off-white crystalline solid
(10.7g)
TLC R_{f} 0.94 (10% methanol in dichloromethane)

### Intermediate 14 Methyl 4-formyl-7-methoxybenzofuran-2-carboxylate

To a suspension of Intermediate 13 (1g) in dichloromethane (14ml) at 0°C was added titanium tetrachloride (1M solution in dichloromethane, 5.3ml) followed by dichloromethyl methyl ether (0.44ml) as a solution in dichloromethane (3ml). The reaction was slowly warmed to room temperature then heated to 35°C for 18h. Upon cooling, the reaction was poured into ice-water and the aqueous phase extracted with dichloromethane. The combined organics were washed with water, dried and concentrated to afford a biscuit coloured solid (0.97g).
TLC R_{f} 0.86 (10% methanol in dichloromethane)

### Intermediate 15 Methyl 4-carboxy-7-methoxybenzofuran-2-carboxylate

To a stirred suspension of Intermediate 14 (0.97g) in t-butanol (115 ml) was added 2-methyl-2-butene (3ml) followed by sodium phosphate (5.7g in 28ml water) and sodium chlorite (3.74g in 28ml water). Stirring was continued for 4h, the reaction solution concentrated and partitioned between ethyl acetate and 10% aqueous hydrochloric acid solution. The organics were dried and concentrated to give a beige solid. Purification by trituration with ether afforded a pale yellow solid (0.62g).
TLC R_{f} 0.64 (10% methanol in dichloromethane)

### Intermediate 16 7-Methoxy-2-methoxycarbonylbenzofuran-4-carbonyl chloride

Intermediate 15 (0.62g) was heated in dry toluene (23ml) with thionyl chloride (2ml) under nitrogen at 90°C for 2h. The solution was evaporated to dryness *in vacuo* and azeotroped with dry toluene (2 x 50ml) to afford the title compound (0.51g) as a pale brown solid.
TLC R_{f} 0.0 (10% methanol in dichloromethane)

### Intermediate 17 2-(1-(t-Butyldimethylsilyloxy)iminoethyl)-7-methoxy-4-[N-(3,5-dichloropyrid-4-yl)]benzofurancarboxamide

To a solution of 2-acetyl-7-methoxy-4-[*N*-(3,5-dichloropyrid-4-yl)]benzofurancarboxamide (0.5g) in toluene (50ml) was added O-(*t*-butyldimethylsilyl)hydroxylamine (0.39g). The reaction mixture was heated under a nitrogen atmosphere under Dean Stark conditions for 3 days then left stirring at room temperature for 2 days. The reaction mixture was concentrated to dryness giving the crude product. Purification by flash chromatography on silica eluting with 50% ethyl acetate in hexane afforded a white solid (0.22g).
TLC R_{f} 0.53 (50% ethyl acetate in hexane)

### Intermediate 18 2-[(Pyridin-4-yl)carbonyl]-7-methoxybenzofuran-4-carbonyl chloride hydrochloride

The title compound was prepared in a similar manner to Intermediate 1.

### Intermediate 19 7-Methoxy-2-[(pyridin-4-yl)carbonyl]benzofuran-4-carboxylic acid

2-[(Pyridin-4-yl)carbonyl]-4-bromo-7-methoxybenzofuran (3.3g), triphenylphosphine (1g), bis(triphenylphosphine)palladium (II) chloride (0.47g), triethylamine (14ml), tetrahydrofuran (150ml) and H₂O (57ml) were combined in a Parr pressure reactor. The vessel was purged with carbon monoxide, charge to 180psi with carbon monoxide and then heated to 80°C with stirring for 3 days. On cooling and release of pressure the tetrahydrofuran was removed *in vacuo* The remaining aqueous mixture was basified to pH14 with 1N NaOH solution (250ml) and washed with ethyl acetate (200ml). The aqueous layer was then acidified to pH5 with acetic acid under ice bath cooling. The resulting precipitate was collected by filtration and dried to give a beige solid (2.97g).
M.S. M+H observed

The following Intermediate was prepared by a similar procedure.

### Intermediate 20 7-Methoxy-2-(2-thiazolocarbonyl)benzofuran-4-carboxylic acid

The title compound was obtained as a cream solid (625mg).
M.S. [M+H] observed

### Intermediate 21 4-Bromo-7-methoxy-2-[(pyridin-4-yl)carbonyl]benzofuran

Bromine (0.02ml) was added to a mixture of 2-[(pyridin-4-yl)carbonyl]-7-methoxy benzofuran (0.1g) in methanol (7ml) under a nitrogen atmosphere cooled to -78°C. The reaction mixture was allowed to warm to room temperature over 2.5hrs. The reaction was then diluted with ethyl acetate (40ml), washed with 5% sodium metabisulfite solution (2x20ml), saturated sodium bicarbonate solution (40ml), dried over MgSO₄ and concentrated to dryness to afford a pale yellow solid (0.05g) as a 2:1 mixture of product:starting material by nmr.
TLC R_{f} 0.65 (10% methanol in ethyl acetate)

### Intermediate 22 4-Bromo-7-methoxy-2-(2-thiazolocarbonyl)benzofuran

A stirred solution of 7-methoxy-2-(2-thiazolocarbonyl)benzofuran (3.09g) in methanol (160ml) was cooled to 0°C under an inert atmosphere and bromine (0.61ml) added dropwise. Stirring was continued for 18h at room temperature and the solvent was then removed *in vacuo*. The residue was partitioned between 5N potassium hydroxide(60ml)/5% sodium metabisulfite (200ml) and ethyl acetate (100ml). The aqueous phase was extracted with ethyl acetate(3 x 60ml), dried (magnesium sulphate) and concentrated *in vacuo* to give the title compound as a beige solid (2.83g).
TLC R_{f} 0.55 (50% ethyl acetate in hexane)

### Intermediate 23 7-Methoxy-2-[(pyridin-4-yl)carbonyl]benzofuran

4-(Bromoacetyl)pyridine hydrobromide (5g) and *o*-vanillin (2.11g) were reacted in a similar manner to Intermediate 11 to give the title compound as a yellow solid (0.95g).
TLC R_{f} 0.53 (ethyl acetate)

### Intermediate 24 7-Methoxy-2-(2-thiazolocarbonyl)benzofuran

To a stirred solution of *o*-vanillin (2.95g) in ethanol (70ml) at 55°C was added sodium hydroxide (1.7g) and stirring continued for 10 minutes. 2-Bromoacetylthiazole hydrobromide (5.57g) was then added portionwise and heating was continued for 5h. The solution was allowed to cool and concentrated *in vacuo.* The residue was partitioned between water (200ml) and ethyl acetate (100ml) and extracted with ethyl acetate (3x70ml), the combined organic phases were dried over magnesium sulphate and concentrated *in vacuo* to give a brown solid. Purification by flash chromatography on silica eluting with 50% ethyl acetate in hexane gave the title compound as orange needles (3.09g).
TLC R_{f} 0.55 (50% ethyl acetate in hexane)

### Intermediate 25 4-(Bromoacetyl)pyridine hydrobromide

4-Acetylpyridine (10g) was combined with 48% HBr solution (18ml) and heated to 70°C. Bromine (4.7ml) dissolved in 48% HBr solution (5ml) was then added dropwise and heating then continued for 2.5hrs. The precipitate which had formed was collected by filtration, washed with 1:1 methanol:hexane (20ml) and dried to give a cream solid (19.5g) as 2:1 mixture of product:starting material by nmr.
mp 170-172°C

The following Intermediate was prepared in a similar manner.

### Intermediate 26 2-Bromoacetylthiazole hydrobromide

The title compound was obtained as a pale yellow solid (5.57g).
¹H NMR (d₆-DMSO) δ 5.00 (2H, CH₂), 8.2 (1H, aromatic), 8.4 (1H, aromatic).

### Intermediate 27 4-Nitrophenyl 7-methoxy-2-(2-thiazolocarbonyl)-benzofuran-4-carboxylate

To a stirred solution of 7-methoxy-2-(2-thiazolocarbonyl)benzofuran-4-carboxylic acid (625mg) in dichloromethane (40ml) were added 1-(3-dimethylaminopropyl)-3-ethylcarbodiimide hydrochloride (593mg), 4-nitrophenol (430mg) and 4-dimethylaminopyridine (catalytic amount). Stirring was continued for 20h, then the precipitate was filtered off, washed with dichloromethane and dried *in vacuo* to give the title compound as a white solid (720mg).
¹H NMR (CDCl₃) δ 4.2 (3H, OCH₃), 7.1(1H, aromatic), 7.6(2H, aromatic), 7.8(1H, aromatic), 8.2(1H, aromatic), 8.3(1H, aromatic), 8.5(2H, aromatic), 9.2(1H, aromatic)

The following Intermediate was prepared by a similar procedure.

### Intermediate 28 4-Nitrophenyl 2-ethyl-7-methoxybenzofuran-4-carboxylate

The title compound (1.26g) was obtained as a white solid.
TLC R_{f} 0.3 (50% ethyl acetate in hexane)

### Intermediate 29 t-Butyl 2-acetyl-7-methoxybenzofuran-4-carboxylate

A solution of 2-acetyl-7-methoxybenzofuran-4-carboxylic acid (100mg) in dichloromethane (4ml) was stirred at room temperature under an atmosphere of nitrogen. *t*-Butyl-2,2,2-trichloroacetimidate (0.16ml) followed by boron trifluoride etherate (0.012ml) were added and the mixture stirred at room temperature for 2h. The reaction was quenched by the addition of a saturated solution of sodium bicarbonate (1ml). The mixture was extracted with dichloromethane (3x10ml) and the combined organic phases dried over magnesium sulphate. Removal of the solvent *in vacuo* and purification by flash chromatography eluting with dichloromethane gave the product as a white solid (130mg).
TLC R_{f} 0.25 (dichloromethane)

### Intermediate 30 (Z)-t-Butyl 2-(1-methoxyiminoethyl)-7-methoxybenzofuran-4-carboxylate

A mixture of (*Z*)-*t*-butyl 2-acetyl-7-methoxybenzofuran-4-carboxylate (0.54g), methoxylamine (0.31g), pyridine (0.46ml) and toluene (50ml) was refluxed under Dean and Stark conditions overnight. The mixture was then cooled and the toluene removed *in vacuo.* The residue was taken up in ethyl acetate (100ml) and washed with water (50ml) then brine (50ml). Drying over magnesium sulphate followed by removal of the solvent *in vacuo* and purification by flash chromatography eluting with dichloromethane gave the product as a colourless oil.
TLC R_{f} 0.52 (dichloromethane)

### Intermediate 31 (Z)-2-(1-Methoxyiminoethyl)-7-methoxybenzofuran-4-carboxylic acid

A solution of (*Z*)-*t*-butyl 2-(1-methoxyiminoethyl)-7-methoxybenzofuran-4-carboxylate (100mg) and trifluoroacetic acid (0.05ml) in dichloromethane (5ml) was stirred at room temperature for 4h. A further aliquot of trifluoroacetic acid (0.1ml) was added and stirring continued overnight. The solvent was removed *in vacuo* and the residue was evapourated *in vacuo* from toluene (2x5ml) and dichloromethane (2x5ml) to remove excess trifluoroacetic acid. The product was obtained as a white solid (72mg).
TLC R_{f} 0.22 (dichloromethane)
mp 233-234°C

### Intermediate 32 (Z)-4-Nitrophenyl 2-(1-methoxyiminoethyl)-7-methoxybenzofuran-4-carboxylate

A solution of (*Z*)-2-(1-methoxyiminoethyl)-7-methoxybenzofuran-4-carboxylic acid (70mg), 4-nitrophenol (41mg), 1-(3-dimethylaminopropyl)-3-ethylcarbodiimide hydrochloride (56mg) and 4-dimethylaminopyridine (catalytic) in dichloromethane (20ml) was stirred at room temperature for 6h under an atmosphere of nitrogen. The reaction mixture was diluted with dichloromethane (20ml) and washed with water (3x20ml). Drying over magnesium sulphate followed by concentration to dryness *in vacuo* gave a pale yellow solid. Purifcation by flash chromatography eluting with dichloromethane gave the product as a white solid (53mg).
TLC R_{f} 0.42 (dichloromethane)
mp 195-196°C

### Example 1 2-Acetyl-7-methoxy-4-[N-(3,5-dichloropyrid-4yl)]benzofurancarboxamide (Method A)

Sodium hydride (0.03g) was added to a solution of 4-amino-3,5-dichloropyridine (0.08g) in anhydrous *N,N*-dimethylformamide (1ml) at room temperature under nitrogen. This stirred mixture was warmed to 60°C for 1 hour before addition of 2-acetyl-7-methoxybenzofuran-4-carbonyl chloride (generated from 2-acetyl-7-methoxybenzofuran-4-carboxylic acid, 0.12g) washed in with anhydrous *N,N*-dimethylformamide (2ml). The brown mixture was heated at 60°C for 4 hours, allowed to cool, poured into water(100ml) and extracted into ethyl acetate (2x50ml). These organic extracts were washed with water (50ml) and saturated brine(50ml) then dried over magnesium sulphate, filtered and evaporated *in vacuo* to give a crude residue (0.17g). Purification by column chromatography on silica eluting with a 20-80% ethyl acetate in hexane gradient afforded a white solid (0.04g).
TLC R_{f} 0.20 (50% ethyl acetate in hexane)
mp 252-254°C

### Example 2 2-Ethyl-7-methoxy-4-[N-(3,5-dichloropyrid-4-yl)]benzofurancarboxamide (Method B)

A suspension of 2-ethyl-7-methoxybenzofuran-4-carboxylic acid (300mg) in dry toluene (50ml) under an inert atmosphere was treated with thionyl chloride (2ml) and heated to reflux for 2 hours. The cooled reaction mixture was evaporated *in vacuo* and the residue azeotroped with dry toluene (2x 10ml) to afford the acid chloride as a white solid (325mg). 4-Amino-3,5-dichloropyridine (230mg) in dry *N,N*-dimethylformamide (20ml) under an inert atmosphere was treated with sodium bis(trimethylsilyl)amine (1.5ml; 1.0M in tetrahydrofuran) at ambient temperature for 30 minutes. The aforementioned solid acid chloride (325mg) was added to this mixture and heated at 50°C for 3 hours then allowed to cool overnight. It was evaporated *in vacuo*, saturated aqueous sodium bicarbonate (50ml) added and extracted into dichloromethane (2x50ml). These extracts were dried over magnesium sulphate, filtered and evaporated *in vacuo* to give a crude residue. Purification by column chromatography on silica eluting with 50% ethyl acetate in hexane afforded a white solid (210mg).
TLC R_{f} 0.15 (25% ethyl acetate in hexane)
mp 199-200°C

### Example 3 2-Acetyl-7-methoxy-4-[N-(pyrid-4-yl)]benzofurancarboxamide (Method C)

A solution of 2-acetyl-7-methoxybenzofuran-4-carbonyl chloride (164mg) in anhydrous dichloromethane (10ml) under nitrogen at 0°C, was treated with 4-aminopyridine (0.07g), triethylamine (0.12g) and 4-dimethylaminopyridine (2mg). This solution was allowed to warm to room temperature and stirred overnight. The reaction mixture was washed with saturated aqueous sodium bicarbonate (10ml), water (10ml) and saturated brine(10ml) then dried over magnesium sulphate, filtered and evaporated *in vacuo* to give a crude residue. Purification by column chromatography on silica eluting with 5% methanol in dichoromethane afforded a pale yellow solid (85mg).
TLC R_{f} 0.27 (5% methanol in dichloromethane)
mp 247-248°C (dec)

### Example 4 2-Acetyl-7-methoxy-4-[N-(pyrid-4-yl)-N-propyl] benzofurancarboxamide

4-[N-(Propylamino)]pyridine (0.08g) was treated with 2-acetyl-7-methoxybenzofuran-4-carbonyl chloride (0.15g) as in method C to afford a pale yellow foam (129mg).
TLC R_{f} 0.57 (5% methanol in dichloromethane)
IR (film); 1292, 1587, 1647, 1685 cm⁻¹

### Example 5 2-Acetyl-7-methoxy-4-[N-(2-chlorophenyl)]benzofurancarboxamide

2-Chloroaniline (0.42ml) was treated with 2-acetyl-7-methoxybenzofuran-4-carbonyl chloride (1g) as in method C. Purification by column chromatography on silica eluting with 50% ethyl acetate in hexane afforded a solid (137mg).
mp 179- 181°C

### Example 6 2-Acetyl-7-methoxy-4-[N-(2,6-dimethylphenyl)]benzofurancarboxamide

2,6-Dimethylaniline (0.49ml) was treated with 2-acetyl-7-methoxybenzofuran-4-carbonyl chloride (1g) as in method C. Purification by column chromatography on silica eluting with 50% ethyl acetate in hexane afforded a solid (255mg).
TLC R_{f} 0.23 (50% ethyl acetate in hexane)
mp 225 - 226°C

### Example 7 2-Acetyl-7-methoxy-4-[N-(4-methoxyphenyl)]benzofurancarboxamide

4-Methoxyaniline (567mg) was treated with 2-acetyl-7-methoxybenzofuran-4-carbonyl chloride (1.19g) as in method C. Purification by column chromatography on silica eluting with 50% ethyl acetate in heptane afforded a yellow solid (103mg).
TLC R_{f} 0.26 (50% ethyl acetate in heptane)

### Example 8 2-Acetyl-7-methoxy-4-[N-(3-bromo-5-methylpyrid-2-yl)]-benzofurancarboxamide (Method D)

2-Amino-3-bromo-5-methylpyridine (0.64g) in dry tetrahydrofuran (20ml) was treated with sodium hydride (0.15g; 60% dispersion in oil) under an inert atmosphere at ambient temperature for 15 minutes. A solution of 2-acetyl-7-methoxybenzofuran-4-carbonyl chloride (0.86g) in dry tetrahydrofuran (10ml) was added and then stirred overnight before evaporation *in vacuo.* Aqueous sodium bicarbonate (50ml) was added and the mixture extracted with ethyl acetate (2x50ml). These extracts were dried over magnesium sulphate, filtered and evaporated *in vacuo.* The crude residue was purified by column chromatography on silica eluting with 50% ethyl acetate in hexane to afford a pale yellow powder (95mg).
TLC R_{f} 0.5 (50% ethyl acetate in hexane)

### Example 9 2-Acetyl-7-methoxy-4-[N-(3-methylphenyl)]benzofurancarboxamide

m-Toluidine (0.42ml) was treated with 2-acetyl-7-methoxybenzofuran-4-carbonyl chloride (1g) as in method C. Purification by column chromatography on silica eluting with 50% ethyl acetate in hexane afforded a yellow solid (200mg).
TLC R_{f} 0.5 (50% ethyl acetate in hexane)
mp 193- 195°C

### Example 10 2-Acetyl-7-methoxy-4-[N-(3,5-dichloropyrid-2-yl)]benzofurancarboxamide

2-Amino-3,5-dichloropyridine (0.758g) was treated with 2-acetyl-7-methoxybenzofuran-4-carbonyl chloride (1.17g) as in method D using *N,N*-dimethylformamide as a cosolvent. Purification by column chromatography on silica eluting with 3% methanol in dichloromethane afforded a yellow solid (13mg).
TLC R_{f} 0.5 (50% ethyl acetate in hexane)

### Example 11 2-Acetyl-7-methoxy-4-[N-(2-methylphenyl)]benzofurancarboxamide

2-Methylaniline (0.21ml) was treated with 2-acetyl-7-methoxybenzofuran-4-carbonyl chloride (0.5g) as in method C. Purification by column chromatography on silica eluting with 50% ethyl acetate in hexane afforded a yellow solid (128mg).
TLC R_{f}0.24 (50% ethyl acetate in hexane)
mp 174- 175°C

### Example 12 2-Acetyl-7-methoxy-4-[N-(4-methoxy-2-methylphenyl)]-benzofurancarboxamide

4-Methoxy-2-methylaniline (0.56ml) was treated with 2-acetyl-7-methoxybenzofuran-4-carbonyl chloride (1.0g) as in method C. Purification by column chromatography on silica eluting with 50% ethyl acetate in hexane afforded a yellow solid (235mg).
TLC R_{f} 0.25 (50% ethyl acetate in hexane)
mp 217-218°C

### Example 13 2-Acetyl-7-methoxy-4-[N-(pyrimidin-4-yl)]benzofurancarboxamide

4-Aminopyrimidine (0.376g) was treated with 2-acetyl-7-methoxybenzofuran-4-carbonyl chloride (1g) as in method C. Purification by column chromatography on silica eluting with a 0-10% methanol in ethyl acetate gradient afforded a yellow solid (0.14g).
TLC R_{f} 0.49 (10% methanol in ethyl acetate)
mp 212 - 214 °C

### Example 14 2-Acetyl-7-methoxy-4-[N-(2-trifluoromethylphenyl)]-benzofurancarboxamide

2-Aminobenzotrifluoride (0.5ml) was treated with 2-acetyl-7-methoxybenzofuran-4-carbonyl chloride (1.0g) as in method D. Purification by column chromatography on silica eluting with 50% ethyl acetate in hexane afforded a yellow solid (0.12g).
mp 164- 166°C

### Example 15 2-Acetyl-7-methoxy-4-[N-(3-chloropyrid-4-yl)]benzofurancarboxamide

4-Amino-3-chloropyridine (0.26g) was treated with 2-acetyl-7-methoxybenzofuran-4-carbonyl chloride (0.5g) as in method A except that the initial anion generation was performed at ambient temperature and using 15-crown-5 (0.90g). Purification by column chromatography on silica eluting with 5% methanol in dichloromethane afforded an off-white solid (0.08g).
TLC R_{f} 0.65 (5% methanol in dichloromethane)
mp 197 - 200°C

### Example 16 2-Acetyl-7-methoxy-4-[N-(2-trifluoromethoxyphenyl)]-benzofurancarboxamide

2-Trifluoromethoxyaniline (0.49g) was treated with 2-acetyl-7-methoxybenzofuran-4-carbonyl chloride (0.7g) as in method C. Purification by column chromatography on silica eluting with 50% ethyl acetate in hexane afforded a yellow solid (0.065g).
TLC R_{f} 0.49 (50% ethyl acetate in hexane)
mp 163 - 165°C

### Example 17 2-Acetyl-7-methoxy-4-[N-(2-ethylphenyl)]benzofurancarboxamide

2-Ethylaniline (0.48g) was treated with 2-acetyl-7-methoxybenzofuran-4-carbonyl chloride (1.0g) as in method C. Purification by column chromatography on silica eluting with 25% ethyl acetate in hexane afforded an off-white solid (310mg).
TLC R_{f} 0.13 (25% ethyl acetate in hexane)
mp 174-175°C

### Example 18 2-Acetyl-7-methoxy-4-[N-(3-methylpyrid-2-yl)]benzofurancarboxamide

2-Amino-3-picoline (0.32ml) was treated with 2-acetyl-7-methoxybenzofuran-4-carbonyl chloride (0.73g) as in method C. Purification by column chromatography on silica eluting with 5% methanol in dichloromethane afforded a yellow solid (0.12g).
TLC R_{f} 0.40 (5% methanol in dichloromethane)

### Example 19 2-Ethyl-7-methoxy-4-[N-(2-chloropyrid-3-yl)]benzofurancarboxamide

3-Amino-2-chloropyridine (0.88g) was treated with 2-ethyl-7-methoxybenzofuran-4-carbonyl chloride (1.8g) as in method A except that the anion generation was performed at ambient temperature for 1.5hours. Purification by flash chromatography on silica eluting with hot ethyl acetate then trituration with diethyl ether afforded a beige solid (0.53g).
TLC R_{f} 0.35 (50% ethyl acetate in hexane)
mp 124 - 125°C

### Example 20 2-Acetyl-7-methoxy-4-[N-(2-methoxyphenyl)]benzofurancarboxamide

o-Anisidine (0.49g) was treated with 2-acetyl-7-methoxybenzofuran-4-carbonyl chloride (1g) as in method C. Purification by column chromatography on silica eluting with 30% ethyl acetate in hexane afforded a yellow solid (160mg).

### Example 21 2-Acetyl-7-methoxy-4-[N-(2-chloropyrid-3-yl)]benzofurancarboxamide

3-Amino-2-chloropyridine (509mg) was treated with 2-acetyl-7-methoxybenzofuran-4-carbonyl chloride (1.0g) as in method D. Purification by column chromatography on silica eluting with 25% ethyl acetate in hexane afforded a yellow solid (205mg).

### Example 22 2-Acetyl-7-methoxy-4-[N-(2-chloro-6-methylphenyl)]-benzofurancarboxamide

2-Chloro-6-methylaniline (0.56g) was treated with 2-acetyl-7-methoxybenzofuran-4-carbonyl chloride (1g) as in method C. Purification by recrystallisation from dichloromethane afforded a brown solid (160mg).
TLC R_{f} 0.4 (5%methanol in dichloromethane)

### Example 23 2-(1-Hydroxyethyl)-7-methoxy-4-[N-(3,5-dichloropyrid-4-yl)]-benzofurancarboxamide

2-Acetyl-7-methoxy-4-[*N*-(3,5-dichloropyrid-4-yl)]benzofurancarboxamide (0.50g) was suspended in dry methanol (20ml) and treated with sodium borohydride (196mg) at ambient temperature. Some external ice cooling was required then stirred overnight. The reaction mixture was poured into water and extracted into ethyl acetate. Evaporation *in vacuo* yielded a solid that was purified by column chromatography using 5% methanol in dichloromethane to afford a white solid (400mg).
TLC R_{f} 0.52 (80% ethyl acetate in heptane)
mp 229 - 231°C

### Example 24 2-[3-(Pyrid-3-yl)-1-oxopropyl]-7-methoxy-4-[N-(3,5-dichloropyrid-4-yl)]benzofurancarboxamide

A solution of 2-acetyl-7-methoxy-4-[*N*-(3,5-dichloropyrid-4-yl)]benzofurancarboxamide (0.40g) in dry *N,N*-dimethylformamide (5ml) under an inert atmosphere was cooled to -10°C and sodium hydride (60%dispersion in oil, 0.11g) added over 30 minutes. After 1 hour at -10°C, 3-picolyl chloride hydrochloride (0.20g) was added and the mixture stirred for a further 2 hours before allowing to warm to room temperature overnight. It was poured into water and extracted into ethyl acetate. These extracts were washed with water and saturated brine then dried over anhydrous magnesium sulphate, filtered and evaporated *in vacuo*. The resultant residue was purified by column chromatography using a 3-10% methanol in dichloromethane gradient then triturated with diethyl ether to yield a beige powder (15.5mg).
TLC R_{f} 0.27 (10% methanol in dichloromethane).

### Example 25 2-(1-Benzyloxyimino)ethyl-7-methoxy-4-[N-(3,5-dichloropyrid-4-yl)]benzofurancarboxamide

2-Acetyl-7-methoxy-4-[*N*-(3,5-dichloropyrid-4-yl)]benzofurancarboxamide (100mg) was refluxed under Dean-Stark conditions in dry toluene (40ml) with dry pyridine (64 µl) and O-benzylhydroxylamine hydrochloride (126mg) under an inert atmosphere. After 2 hours the mixture was allowed to cool and left stirring overnight. Addition of methanol and acetone formed a precipitate. This was filtered off to afford a solid (26mg).
TLC R_{f} 0.45 (50% ethyl acetate in hexane).

### Example 26 2-Ethyl-7-methoxy-4-[N-(3-carboxyphenyl)]benzofurancarboxamide

A solution of 2-ethyl-7-methoxy-4-[*N*-(3-carboethoxyphenyl)]-benzofurancarboxamide (0.78g) in THF (25ml) was treated with a solution of lithium hydroxide monohydrate (0.18g) in water (25ml) and the reaction mixture stirred at room temperature overnight. The reaction mixture was concentrated *in vacuo*, diluted with water (100ml) and acidified with dilute aqueous hydrochloric acid. The resulting white precipitate was collected, washed with water and dried *in vacuo* to afford the title compound (0.68g) as a white solid.
TLC R_{f} 0.35 (5% methanol in dichloromethane)
mp 265-267°C

The following compound was prepared according to the above procedure:

### Example 27 2-Ethyl-7-methoxy-4-[N-(4-carboxyphenyl)]benzofurancarboxamide

Prepared from 2-ethyl-7-methoxy-4-[*N*-(4-carboethoxyphenyl)]-benzofurancarboxamide (0.67g) to afford the title compound (0.59g) as a white solid.
TLC R_{f} 0.4 (5% methanol in dichloromethane)
mp 279-280°C

### Example 28 2-[1-(2,2-Dimethylpropyl)]-7-methoxy-4-[N-(3,5-dichloropyrid-4-yl)]benzofurancarboxamide

Thionyl chloride (1.65ml) was added to a suspension of 2-[1-(2,2-dimethylpropyl)]-7-methoxybenzofurancarboxylic acid (0.59g) in toluene (10ml) and the mixture heated at reflux for 3h. The mixture was stirred at room temperature overnight and concentrated *in vacuo*. The residue was azeotroped several times with toluene to furnish 2-[1-(2,2-dimethylpropyl)]-7-methoxybenzofuran-4-carbonyl chloride (0.63g). Sodium hexamethyldisilazide (1M solution in THE, 4.5ml) was added to a solution of4-amino-3,5-dichloroaminopyridine (0.74g) in dry DMF (2ml) at room temperature under nitrogen. The mixture was stirred at room temperature for 0.5h, then warmed to 50°C. A solution of 2-[1-(2,2-dimethylpropyl)]-7-methoxybenzofuran-4-carbonyl chloride (0.63g) in DMF was added and the reaction mixture stirred for a further 3h, then at room temperature for 16h. Water (20ml) was added and the resultant precipitate was collected and dried *in vacuo*. Purification by column chromatography on silica, eluting with 25% ethyl acetate in hexane afforded the title compound (0.29g) as a pale yellow solid.
TLC R_{f} 0.4 (50% ethyl acetate in hexane)
mp 164-165°C

### Example 29 2-(1-Methoxyiminoethyl)-7-methoxybenzofuran-4-[N-3,5-dichloropyrid-4-yl)]carboxamide

To a suspension of 2-acetyl-7-methoxy-4-[N-(3,5-dichloropyrid-4-yl)]benzofurancarboxamide (0.07g) in dry toluene (40ml) was added pyridine (0.09ml) and methoxylamine hydrochloride (0.056g) and the mixture heated under Dean-Stark conditions for 24h. Evaporation of the solvent and purification by flash chromatography on silica eluting with dichloromethane then 5% methanol in dichloromethane followed by preparative tlc (5% methanol in dichloromethane) afforded a white solid (0.03g) as a mixture of E and Z isomers by nmr.
TLC R_{f} 0.27-0.34 (50% ethyl acetate in hexane)

### Example 30 N-(3,5-Dichloropyrid-4-yl)-2-carboxy-7-methoxybenzofuran-4-carboxamide

4-Amino-3,5-dichloropyridine (0.31g) was treated with methyl 7-methoxy-2-methoxycarbonylbenzofuran-4-carbonyl chloride as in Example 19. Purification by flash chromatography on silica eluting with 10% methanol in dichloromethane afforded a pale yellow solid (0.15g).
TLC R_{f} 0.1 (10% methanol in dichloromethane)
mp greater that 350°C

### Example 31 2-Ethyl-7-methoxy-4-[N-(2,5-dimethylpyrid-4-yl)]benzofurancarboxamide

4-Amino-2,5-dimethylpyridine (0.3g) was treated with 2-ethyl-7-methoxybenzofuran-4-carbonyl chloride (0.59g) as in Example 19. Purification by flash chromatography on silica eluting with 10% methanol in dichloromethane then trituration with hexane afforded an off-white solid (0.11g)
TLC R_{f} 0.33 (10% methanol in dichloromethane)
mp 164-165°C

### Example 32 N-(5-Chloropyrimidin-4-yl)-2-ethyl-7-methoxybenzofuran-4-carboxamide

4-Amino-5-chloropyrimidine (0.25g) was treated with 2-ethyl-7-methoxybenzofuran-4-carbonyl chloride (0.46g) as in Method B. Purification by recrystallisation afforded an off-white solid (0.12g)
TLC R_{f} 0.25 (3:2 Ethyl Acetate:Hexane)
mp 161-163°C

### Example 33 2-Ethyl-7-methoxy-4-[N-(3-methylthiotriazin-5-yl)]-benzofurancarboxamide

5-Amino-3-methylthiotriazine (0.3g) was treated with 2-ethyl-7-methoxybenzofuran-4-carbonyl chloride (0.50g) as in Example 19. Purification by flash chromatography on silica eluting with 50% ethyl acetate in hexane afforded a cream solid (0.1g)
TLC R_{f} 0.47 (50% ethyl acetate in hexane)
M.S. [M+H] observed

### Example 34 2-Ethyl-7-methoxy-4-[N-(4-aminopyrido[3,2-b]pyridinyl)]-benzofurancarboxamide

4-Aminopyrido[3,2-b]pyridine (0.36g) was treated with 2-ethyl-7-methoxybenzofuran-4carbonyl chloride (0.59g) as in Example 21. Purification by flash chromatography on silica eluting with 10% methanol in ethyl acetate followed by trituration with hexane afforded a pale yellow solid (0.23g)
TLC R_{f} 0.52 (10% methanol in ethyl acetate)
mp 155-157°C

### Example 35 2-Ethyl-7-methoxy-4-[N-(3-fluoropyridin-4-yl)]benzofurancarboxamide

4-Amino-3-fluoropyridine (0.25g) was treated with 2-ethyl-7-methoxybenzofuran-4-carbonyl chloride (0.53g) as in Method B. Purification by flash chromatography on silica eluting with 50% ethyl acetate in hexane afforded an off-white solid (0.13g)
TLC R_{f} 0.15 (50% ethyl acetate in hexane)
mp 150-151°C

### Example 36 Z-2-(1-Hydroxyiminoethyl)-4-[N-(3,5-dichloropyridin-4-yl)]-7-methoxybenzofurancarboxamide

To a suspension of 2-acetyl-7-methoxy-4-[N-(3,5-dichloropyrid-4-yl)]benzofuran carboxamide (0.5g) in dry toluene (30ml) were added pyridine (1g) and hydroxylamine (0.9g) and the mixture heated under Dean-Stark conditions for 48h. Evaporation of the solvent and washing with water afforded an off-white solid (0.2g)
TLC R_{f} 0.22 (50% ethyl acetate in hexane)
mp 250°C (decomp)

### Example 37 2-Ethyl-7-methoxy-4-[N-(2-chloro-5-carboxyl)phenyl]-benzofurancarboxamide

5-Carboxymethyl-2-chloroaniline (0.566g) was treated with 2-ethyl-7-methoxybenzofuran-4-carbonyl chloride (0.50g) as in Example 19. Purification by flash chromatography on silica eluting with 50% ethyl acetate in hexane afforded 2-ethyl-7-methoxy-4-[N-(2-chloro-5-methoxycarbonyl)phenyl]benzofurancarboxamide as a white solid (0.497g; TLC R_{f} 0.5 (50% ethyl acetate in hexane); mp 174-176°C). This product (0.31 g) was treated as in Example 26, to afford the title compound (0.282g) as a white solid.
TLC R_{f} 0.6 (ethyl acetate)
mp 278-279°C

### Example 38 Z-2-(1-Hydroxyiminoethyl)-4-[N-methyl-N-(3,5-dichloropyridin-4-yl)]-7-methoxybenzofurancarboxamide

To a solution of Z-2-(1-hydroxyiminoethyl)-4-[N-(3,5-dichloropyridin-4-yl)]-7-methoxybenzofurancarboxamide (50mg) in tetrahydrofuran (10ml) was added Bu₄NI (cat. amount) followed by a solution of NaOH (6mg) in H₂O (1ml). The whole was stirred for 20mins then MeI (45mg) was added and stirring continued for 12hrs. The mixture was concentrated to dryness, diluted with EtOAc (20ml), washed with H₂O (10ml), brine (10ml), dried over MgSO₄ and concentrated to dryness again giving the crude product. Purification by flash chromatography on silica eluting with 50% ethyl acetate in hexane afforded a white solid (24mg).
TLC R_{f} 0.37 (50% ethyl acetate in hexane)
mp 97-98°C

### Example 39 Z-2-(1-(4-Pyridylmethoxy)iminoethyl)-7-methoxybenzofuran-4-[N(3,5-dichloropyrid-4-yl)]carboxamide

To a solution ofZ-2-(1-hydroxyiminoethyl)-4-[N-(3,5-dichloropyridin-4-yl)]-7-methoxybenzofurancarboxamide (50mg) in tetrahydrofuran (10ml) was added Bu₄NI (cat. amount) followed by a solution of NaOH (17mg) in H₂O (1ml). The whole was stirred for 20mins then 4-chloromethylpyridine hydrochloride (46mg) was added and stirring continued for 48hrs. The mixture was concentrated to dryness, diluted with EtOAc (20ml), washed with H₂0 (10ml), brine (10ml), dried over MgSO₄ and concentrated to dryness again giving the crude product. Purification by flash chromatography on silica eluting with 50% ethyl acetate in hexane afforded an off-white solid (10mg).
TLC R_{f} 0.26 (ethyl acetate)
mp 217-218°C

### Example 40 2-(1-Hydroxyiminoethyl)-4-[N-(3,5-dichloropyridin-4-yl)]-7-methoxybenzofurancarboxamide

Tetrabutylammonium fluoride (1M solution in tetrahydrofuran) (0.48ml) was added to a solution of 2-(1-(t-butyldimethylsilyloxy)iminoethyl)-7-methoxy-4-[N-3,5-dichloropyrid-4-yl]benzofurancarboxamide in tetrahydrofuran (10ml) under a nitrogen atmosphere. Stirring was continued for 20 mins then the reaction mixture was concentrated to dryness giving the crude product. Purification by flash chromatography on silica eluting with 50% ethyl acetate in hexane afforded an off-white solid (0.1g) as a 2:1 mixture of E:Z isomers by nmr.
TLC R_{f} 0.22 (50% ethyl acetate in hexane)
mp 280°C (dec.)

### Example 41 2-[(Pyridin-4-yl)carbonyl]-7-methoxybenzofuran-4-[N-(3,5-dichloropyridin-4-yl)]carboxamide

Sodium hydride (0.3g) was added to a solution of 4-amino-3,5-dichloropyridine (0.56g) in dimethylformamide (10 ml) under an atmosphere of nitrogen. The reaction mixture was heated to 55°C for 1hr, then 2-[(pyridin-4-yl)carbonyl]-7-methoxybenzofuran-4-carbonyl chloride was added in one portion. Heating at 55°C was continued for 2hrs then at room temperature for 12hrs. The reaction mixture was concentrated to dryness to give the crude product. Purification by flash chromatography on silica eluting with ethyl acetate and then 20% methanol in ethyl acetate afforded a cream solid (0.3g). TLC R_{f} 0.36 (ethyl acetate)
mp 250-252°C

### Example 42 2-[Methoxyimino(4-pyridyl)methyl]-7-methoxybenzofuran-4-[N-(3,5dichloropyridin-4-yl)]carboxamide

2-[(Pyridin-4-yl)carbonyl]-7-methoxybenzofuran-4-[N-(3,5-dichloropyridin-4-yl)]-carboxamide (0.25g) was treated with methoxylamine hydrochloride (0.165g) as in Example 29. The crude product was washed with H₂O and then diethyl ether to give a white solid (0.217g) as a 5.5:4.5 mixture of E:Z isomers by nmr.
mp 245-247°C
M.S. [M+H] observed

### Example 43 E-2-(1-(4-Pyridylmethoxy)iminoethyl)-7-methoxybenzofuran-4-[N(3,5-dichloropyrid-4-yl)]carboxamide

Sodium (9mg) was added to a suspension of 2-(1-hydroxyiminoethyl)-4-[N-(3,5-dichloropyridin-4-yl)]-7-methoxybenzofurancarboxamide (50mg) in ethanol (2ml) under a nitrogen atmosphere. Once all solids had gone into solution 4-chloromethylpyridine hydrochloride (21mg) was added and the reaction mixture was stirred at room temperature for 3 days. More sodium (6mg) and 4-chloromethylpyridine hydrochloride (21mg) were added and the reaction mixture was heated to 85°C for 6hrs. The reaction was quenched with H₂O (20ml), extracted with ethyl acetate (3 x 20ml). The combined organic layers were washed with H₂O (20ml), brine (20ml), dried over MgSO₄ and concentrated to dryness to give the crude product. Purification by flash chromatography on silica eluting with ethyl acetate afforded an off-white solid (18mg).
TLC R_{f} 0.43 (ethyl acetate)
mp 231-232°C

### Example 44 2-(4-Morpholino)acetyl-7-methoxybenzofuran-4-[N-(3,5-dichloropyrid-4-yl)carboxamide

Bromine (0.01ml) was added to a solution of 2-acetyl-7-methoxy-4-[N-(3,5-dichloropyridin-4-yl)]benzofurancarboxamide (0.1g) in 45% HBr/AcOH (4ml) under an atmosphere of nitrogen. The reaction mixture was stirred at room temperature for 12hrs then quenched with H₂O (20ml) and extracted with ethyl acetate (3 x 20ml). The combined organic layers were washed with saturated aqueous sodium bicarbonate (20ml), brine (20ml), dried over MgSO₄ and concentrated to dryness to give the crude 2-bromoacetyl-7-methoxybenzofutan-4-[N-(3,5-dichloropyrid-4-yl)]carboxamide. Purification by flash chromatography on silica eluting with 50% ethyl acetate in hexane afforded an impure yellow solid (20mg) which was used without further purification.

Morpholine (0.09ml) and triethylamine (10mg) were added to a solution of 2-bromoacetyl-7-methoxybenzofuran-4-[N-(3,5-dichloropyrid-4-yl)]carboxamide (20mg) in dichloromethane (5ml) under a nitrogen atmosphere. Stirring was continued for 1.5hrs. The reaction mixture was then diluted with more dichloromethane washed with H₂O, dried over MgSO₄ and concentrated to dryness to give the crude product. Purification by flash chromatography on silica eluting with ethyl acetate afforded a yellow solid (10mg).
TLC R_{f} 0.38 (ethyl acetate)
mp 130°C (dec.)

### Example 45 N-(2,6-Dichloro-4-carboxyphenyl)-2-ethyl-7-methoxybenzofuran-4-carboxamide

Ethyl 4-amino-3,5-dichlorobenzoate (0.815g) was treated with 2-ethyl-7-methoxybenzofuran-4-carbonyl chloride (0.754g) as in Example 19. Purification by triturating the crude product with dichloromethane afforded N-[2,6-dichloro-4-(ethoxycarbonyl)phenyl]-2-ethyl-7-methoxybenzofuran-4-carboxamide a white solid (338mg; TLC R_{f} 0.15 (20% ethyl acetate in hexane); mp 165-166°C). This product (292 mg) was treated as in Example 26 to afford the title compound (230mg) as a white solid.
TLC R_{f} 0.6 (6% methanol in dichloromethane)
mp 274-275°C

### Example 46 7-Methoxy-2-(2-thiazolocarbonyl)-4-[N-(5-chloropyrimidin-4-yl)]benzofurancarboxamide

To a stirred solution of 4-amino-5-chloropyrimidine (220mg) in dimethylformamide (20ml) under nitrogen was added sodium hydride (60% dispersion in oil) (135mg) and stirring was continued for 3h. 4-Nitrophenyl 7-methoxy-2-(2-thiazolocarbonyl)-benzofuran-4-carboxylate (720mg) was then added and stirring was continued for a further 18 h. The solvent was removed *in vacuo* and the resulting residue was triturated with ethyl acetate then purified by flash chromatography eluting with 2% ammonium hydroxide/20% methanol in ethyl acetate. Further trituration with methanol yielded the title compound as a cream solid (165mg).
M.S. [M⁺H] observed
mp 262-264°C (dec)

The following examples were prepared from 4-nitrophenyl 2-ethyl-7-methoxybenzofuran-4-carboxylate and the appropriate amine according to the above procedure.

### Example 47 2-Ethyl-7-methoxy-4-[(N-(2,5-difluoropyrimidin-4-yl)]benzofurancarboxamide

Prepared from 4-amino-2,5-difluoropyrimidine (190mg) to give the title compound (95mg) as an off-white solid.
TLC R_{f} 0.6 (50% ethyl acetate in hexane)
mp 175-176°C

### Example 48 2-Ethyl-7-methoxybenzofuran-4-[(N-(1,3,5-trimethylpyrazol-4-yl)]carboxamide

Prepared from 4-amino-1,3,5-trimethylpyrazole (165mg) to give the title compound (222mg) as a white solid.
TLC R_{f} 0.27 (10% methanol in ethyl acetate)
mp 182-184°C

### Example 49 2-[2-(4-Morpholino)-(2-methoxy)iminoethyl]-7-methoxy-4-[N-(3,5-dichloropyrid-4-yl)]benzofurancarboxamide

Prepared from 2-(4-morpholino)acetyl-7-methoxy-4-[*N*-(3,5-dichloropyrid-4-yl)]benzofurancarboxamide by a similar procedure to that of Example 4. Purification by flash chromatography on silica eluting with ethyl acetate afforded the product as a yellow solid as a 1:1 mixture of isomers (20mg).
TLC R_{f} 0.66 and 0.53 (ethyl acetate)
mp 140°C (dec.)

### Example 50 (Z)-2-[1-(2-Methylthiazol-4-ylmethoxy)iminoethyl]-7-methoxy-4-[N-(3,5-dichloropyrid-4-yl)]benzofurancarboxamide

A solution of (*Z*)-2-(1-hydroxyiminoethyl)-7-methoxy-4-[*N*-(3,5-dichloropyridin-4-yl)]benzofurancarboxamide (0.75g) in dimethylformamide (30ml) was stirred at room temperature under an atmosphere of nitrogen. Sodium hydride (60% dispersion in oil) (0.17g) was added and stirring continued for 1h. 4-Chloromethyl-2-methylthiazole (0.84g) was added (generated from the hydrochloride salt using sodium bicarbonate) and the mixture stirred for 1h. The mixture was poured onto water (100ml) and extracted with ethyl acetate (3x100ml). The combined organic washings were washed with water (100ml) and brine (50ml), dried over magnesium sulphate and the solvent removed *in vacuo* to give creamy solid. The solid was triturated with ether to removed unreacted alkylating agent and purified by flash chromatography eluting with 0-10% methanol in ethyl acetate to give the product as a white solid (0.69g).
TLC R_{f} 0.62 (ethyl acetate)
mp 221-222°C

The following Example was prepared by a similar procedure.

### Example 51 (Z)-2-(1-(4-Morpholinoethoxy)iminoethyl)-7-methoxy-4-[N-(3,5-dichloropyrid-4-yl)]benzofurancarboxamide

Purification by recrystallisation from ethyl acetate/hexane gave the product as a white solid (0.22g)
TLC R_{f} 0.50 (10% methanol in dichloromethane)
mp 178-179°C

### Example 52 (Z)-2-(1-Methoxyiminoethyl)-7-methoxy-4-[N-(3,5-dichloropyrid-4-yl)]benzofurancarboxamide

A solution of 4-amino-3,5-dichloropyidine (23mg) in dimethylformamide (5ml) was stirred at room temperature under an atmosphere of nitrogen. Sodium hydride (60% dispersion in oil) (9mg) was added and the resulting suspension stirred for 1h. A solution of 4-nitrophenyl 2-(1-methoxyiminoethy)]-7-methoxybenzofuran-4-carboxylate (50mg) in dimethylformamide (2ml) was added and stirring continued overnight The reaction was quenched by the addition of water (1ml) and the mixture concentrated to dryness *in vacuo*. Purification by flash chromatography eluting with 50% ethyl acetate in hexane gave the product as a white solid (23mg).
TLC R_{f} 0.40 (50% ethyl acetate in hexane)
mp 238-239°C

### Assay methods

The assays used to confirm the phosphodiesterase IV inhibitory activity of compounds of formula (i) are standard assay procedures as disclosed by Schilling *et al*, Anal. Biochem. 216:154 (1994), Thompson and Strada, Adv. Cycl. Nucl. Res. 8:119 (1979) and Gristwood and Owen, Br. J. Pharmacol. 87:91P (1986).

Compounds of formula (i) have exhibited activity at levels consistent with those believed to be useful in treating phosphodiesterase IV-related disease states in those assays.

The ability of compounds of formula (i) to inhibit TNF production in human peripheral blood mononuclear cells (PBMC's) is measured as follows. PBMC's are prepared from freshly taken blood or "Buffy coats" by standard procedures. Cells are plated out in RPMI1640 +1% foetal calf serum in the presence and absence of inhibitors. LPS (100 ng/ml) is added and cultures are incubated for 22 h at 37°C in an atmosphere of 95% air/5% CO₂. Supernatants are tested for TNFα by ELISA using commercially available kits.

*In vivo* activity in a skin eosinophilia model is determined by using the methods described by Hellewell *et al*, Br. J. Pharmacol. 111:811 (1994) and Br. J. Pharmacol. 110:416 (1993). Activity in a lung model is measured using the procedures described by Kallos and Kallos, Int. Archs. Allergy Appl. Immunol. 73:77 (1984), and Sanjar *et al*, Br. J. Phannacol. 99:679 (1990).

An additional lung model, which allows measurement of inhibition of the early and late-phase asthmatic responses and also the inhibition of airway hyperreactivity, is described by Broadley *et al*, Pulmonary Pharmacol. 7:311 (1994), J. Immunological Methods 190:51 (1996) and British J. Pharmacol. 116:2351 (1995). Compounds of the invention show activity in this model.

### Abbreviations

- LPS: Lipopolysaccharide (endotoxin)
- ELISA: Enzyme linked immunosorbent assay

## Claims

1. Use of a compound of the general formula (i) 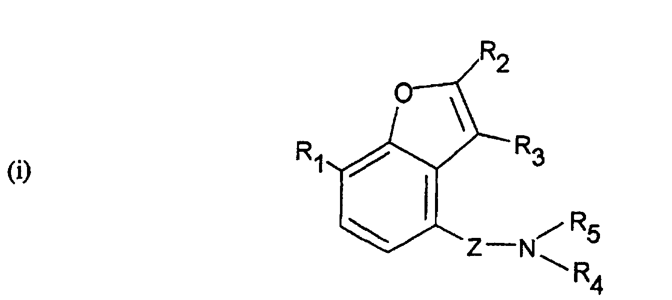 wherein Z is CO or CS;
R₁ represents alkoxy optionally substituted with one or more halogens, OH or thioalkyl;
R₂ and R₃ are the same or different and are each H, R₆, OR₁₀, COR₆, C(=NOR₆)R₆, alkyl-C(=NOR₆)R₆, alkyl-C(=NOH)R₆, C(=NOH)R₆, halogen, NR₈R₉, CF₃, CN, CO₂H, CO₂R₁₀, CONH₂, CONHR₆ or CON(R₆)₂;
R₄ represents H, arylalkyl, heteroarylalkyl, heterocycloalkyl, S(O)ₘR₁₀ or alkyl optionally substituted with one or more substituents chosen from hydroxy, alkoxy, CO₂R₇, SO₂NR₁₁R₁₂, CONR₁₁R₁₂, CN, carbonyl oxygen, NR₈R₉, COR₁₀ and S(O)ₙR₁₀;
R₅ represents aryl, heteroaryl, heterocyclo, arylalkyl, heteroarylalkyl or heterocycloalkyl;
in R₄ and/or R₅ the aryl/heteroaryl/heterocyclo portion is optionally substituted with one or more substituents alkyl-R₁₃ or R₁₃;
R₆ represents R₁₀ optionally substituted at any position with R₁₄;
R₇ represents H, alkyl, arylalkyl, heteroarylalkyl or heterocycloalkyl;
R₈ represents H, aryl, heteroaryl, heterocyclo, alkyl, cycloalkyl, arylalkyl, heteroarylalkyl, heterocycloalkyl, alkylcarbonyl, alkoxycarbonyl, arylsulphonyl, heteroarylsulphonyl, heterocyclosulphonyl, arylcarbonyl, heteroarylcarbonyl, heterocyclocarbonyl or alkylsulphonyl;
R₁₀ represents alkyl, cycloalkyl, aryl, heteroaryl, heterocyclo, arylalkyl, heteroarylalkyl or heterocycloalkyl;
R₉, R₁₁ and R₁₂ are the same or different and are each H or R₁₀;
R₁₃ represents alkyl optionally substituted with halogen, alkoxy optionally substituted by halogen, aryl, heteroaryl, heterocyclo, hydroxy, aryloxy, heteroaryloxy, heterocyclooxy, arylalkyloxy, heteroarylalkyloxy, heterocycloalkyloxy, CO₂R₇, CONR₁₁R₁₂, SO₂NR₁₁R₁₂, halogen, -CN, -NR₈R₉, COR₁₀, S(O)ₙR₁₀ or carbonyl oxygen;
R₁₄ represents OH, carbonyl oxygen, OR₁₀, NR₈R₉, CN, CO₂H, CO₂R₁₀, CON₁₁R₁₂ or COR₁₀;
m is an integer of up to 2; and
n represents 0-2;
or a pharmaceutically-acceptable salt thereof,
for the manufacture of a medicament for the treatment of a disease state selected from chronic bronchitis, chronic obstructive airways disease, atopic dermatitis, urticaria, allergic conjunctivitis, vemal conjunctivitis, inflammation of the eye, allergic responses in the eye, eosinophilic granuloma, gouty arthritis, ulcerative colitis, adult respiratory distress syndrome, diabetes insipidus, keratosis, atopic eczema, cerebral senility, multi-infarct dementia, senile dementia, memory impairment associated with Parkinson's disease, depression, cardiac arrest, stroke, intermittent claudication, joint inflammation, osteoarthritis, sepsis, septic shock, endotoxic shock, gram negative sepsis, toxic shock syndrome, acute respiratory distress syndrome, cerebral malaria, chronic pulmonary inflammatory disease, pulmonary sarcoidosis, bone resorption diseases, reperfusion injury, graft *vs* host reaction, allograft rejection, malaria, myalgias, ARC, cachexia, pyresis, Bechet's disease, anaphylactoid purpura nephritis, chronic glomerulonephritis, inflammatory bowel disease, leukemia, a yeast or fungal infection, brain trauma, stroke, ischaemia, Huntingdon's disease or tardive dyskinesia.

2. Use of a compound of formula (i) as defined in claim 1, for the manufacture of a medicament for use in gastroprotection.

3. Use of a compound of formula (i) as defined in claim 1, for the manufacture of a medicament for use as an analgesic, anti-tussive or anti-hyperalgesic in the treatment of neurogenic inflammatory disease associated with irritation and pain.

4. A compound of formula (i) as defined in claim 1, with the proviso that the following compounds are excluded, i.e. those wherein:
R₁ is optionally substituted alkoxy;
R₂ and R₃ are the same or different and are each H, alkyl, cycloalkyl, phenyl, naphthyl or heteroaryl (wherein phenyl, naphthyl or heteroaryl is optionally substituted by 1 to 3 substituents selected from OH, alkoxy, alkanoyl, alkoxycarbonyl, NH₂ and CN), alkanoyl, cycloalkanoyl, alkoxycarbonyl, CN, -(CH₂)₁₋₄-COOR₁₀ when R₁₀ is H, alkyl, cycloalkyl, phenyl, naphthyl, heterocyclo or arylalkyl, or -(CH₂)₁₋₄-CONR₁₁R₁₂ when R₁₁ and R₁₂ are each H, alkyl, cycloalkyl, phenyl, naphthyl, heteroaryl or arylalkyl;
R₄ is H, alkyl or cycloalkyl; and
R₅ is 4-pyridyl optionally substituted at the 3 and/or 5 positions by alkyl, OH, alkoxy, COOH, alkanoyl, alkoxy-CO-, CF₃, NH₂, CN, NO₂ or halogen.

5. A compound of claim 4, wherein R₂ and R₃ are the same or different and are each H, R₆, COR₆, C(=NOR₆)R₆, CN, CO₂H, CO₂R₁₀, CONH₂, CONHR₆ or CON(R₆)₂.

6. A compound of claim 4, wherein
Z is CO;
R₁ is alkoxy optionally substituted by one or more halogens;
R₂ and R₃ are the same or different and are each R₆ₐ or alkyl-R₆ₐ;
R₆ₐ is H, aryl, heteroaryl, heterocyclo, hydroxy, alkoxy, aryloxy, heteroaryloxy, heterocyclooxy, arylalkyloxy, heteroarylalkyloxy, heterocycloalkyloxy, alkylamino, CF₃ or COR₁₀;
R₁₀ is alkyl, aryl, heteroaryl, heterocyclo, arylalkyl, heteroarylalkyl or heterocycloalkyl;
R₁₃ is aryl, heteroaryl, heterocyclo, hydroxy, alkoxy, aryloxy, heteroaryloxy, heterocyclooxy, arylalkoxy, heteroarylalkyloxy, heterocycloalkyloxy, CO₂R₇, CONR₁₁R₁₂, SO₂NR₁₁R₁₂, halogen, CN, NR₈R₉, COR₁₀, S(O)ₙR₁₀ or carbonyl oxygen; and
m is 1 or 2.

7. A compound of claim 4, wherein
R₂ and R₃ are each independently selected from H, R₆ and COR₆; and
R₆ is alkyl, aryl, heteroaryl, heterocyclo, arylalkyl, heteroarylalkyl or heterocycloalkyl.

8. A compound of any of claims 4 to 7, wherein R₂ is not H.

9. A compound of any of clairns 4 to 8,wherein R₅ is optionally-substituted aryl or heteroaryl.

10. A compound of any of claims 4 to 9, wherein R₁ is alkoxy optionally substituted with one or more halogens.

11. A compound of any of claims 4 to 10, wherein R₂ and R₃ are not both H.

12. A compound of any of claims 4 to 11, wherein R₂ and R₃ are independently OR₁₀, halogen, NR₈R₉ or CF₃.

13. A compound of any of claims 4 to 11, wherein R₂ and R₃ are independently C(=NOR₆)R₆, alkyl-C-(=NOR₆)R₆, C(=NOH)R₆ or alkyl-C-(=NOH)R₆.

14. A compound of any of claims 4 to 11, wherein R₂ and R₃ are independently heterocyclo, heterocycloalkyl or heteroarylalkyl optionally substituted at any position with (one or more) R₁₄.

15. A compound of any of claims 4 to 11, wherein R₂ and R₃ are independently alkyl substitution at any position with (one or more) OH, OR₁₀, NR₈R₉ or CN.

16. A compound of any of claims 4 to 11, wherein R₂ and R₃ are independently alkyl substituted with one or more COR and R is aryl, heteroaryl, heterocyclo (not attached through nitrogen), arylalkyl, heteroarylalkyl or heterocycloalkyl.

17. A compound of any of claims 4 to 11, wherein R₂ and R₃ are independently COR as defined in claim 16 optionally substituted at any position with (one or more) R₁₄.

18. A compound of any of claims 4 to 17, wherein R₄ is H or alkyl.

19. A compound of any of claims 4 to 18, wherein R₅ is aryl or heteroaryl, either of which may be optionally substituted with one or more substituents alkyl-R₁₃ or R₁₃.

20. A compound of claim 4, selected from
2-acetyl-7-methoxy-4-*N*-(2-chlorophenyl)benzofurancarboxamide,
2-acetyl-7-methoxy-4-*N*-(2,6-dimethylphenyl)benzofurancarboxamide,
2-acetyl-7-methoxy-4-*N*-(4-methoxyphenyl)benzofurancarboxamide,
2-acetyl-7-methoxy-4-*N-*(3-bromo-5-methylpyrid-2-yl)benzofurancarboxamide,
2-acetyl-7-methoxy-4-*N*-(3-methylphenyl)benzofurancarboxamide,
2-acetyl-7-methoxy-4-*N*-(3,5-dichloropyrid-2-yl)benzofurancarboxamide,
2-acetyl-7-methoxy-4-*N*-(2-methylphenyl)benzofurancarboxamide,
2-acetyl-7-methoxy-4-*N*-(4-methoxy-2-methylphenyl)benzofurancarboxamide,
2-acetyl-7-methoxy-4-*N*-(pyrimidin-4-yl)benzofurancarboxamide,
2-acetyl-7-methoxy-4-*N*-(2-trifluoromethylphenyl)benzofurancarboxamide,
2-acetyl-7-methoxy-4-*N*-[2-(piperidin-1-yl)phenyl]benzofurancarboxamide,
2-acetyl-7-methoxy-4-*N*-(3-chloropyrid-4-yl)benzofurancarboxamide,
2-acetyl-7-methoxy-4-*N*-(2-trifluoromethoxyphenyl)benzofurancarboxamide,
2-acetyl-7-methoxy-4-*N*-(2-ethylphenyl)benzofurancarboxamide,
2-acetyl-7-methoxy-4-*N*-(2-biphenyl)benzofurancarboxamide,
2-acetyl-7-methoxy-4-*N*-(3-methylpyrid-2-yl)benzofurancarboxamide,
2-ethyl-7-methoxy-4-*N*-(2-chloropyrid-3-yl)benzofurancarboxamide,
2-acetyl-7-methoxy-4-*N*-(2-methoxyphenyl)benzofurancarboxamide,
2-acetyl-7-methoxy-4-*N*-(2-chloro-6-methylphenyl)benzofurancarboxamide,
2-(1-hydroxyethyl)-7-methoxy-4-*N*-(3,5-dichloropyrid-4-yl)benzofurancarboxamide,
2-(3-pyrid-3-yl-1-oxopropyl)-7-methoxy-4-*N*-(3,5-dichloropyrid-4-yl)benzofurancarboxamide,
2-(1-benzyloxyimino)ethyl-7-methoxy-4-*N*-(3,5-dichloropyrid-4-yl)benzofurancarboxamide,
2-ethyl-7-methoxy-4-*N*-(3-carboxyphenyl)benzofurancarboxamide,
2-ethyl-7-methoxy-4-*N*-(4-carboxyphenyl)benzofurancarboxamide, and
2-(2,2-dimethylpropyl)-7-methoxy-4-*N*-(3,5-dichloropyrid-4-yl)benzofurancarboxamide.

21. A compound selected from
2-acetyl-7-methoxy-4-*N*-(3,5-dichloropyrid4-yl)benzofurancarboxamide and
2-acetyl-7-methoxy-4-*N*-(pyrid-4-yl)-benzofurancarboxamide;
or a pharmaceutically-acceptable salt thereof.

22. A compound selected from
2-ethyl-7-methoxy-4-*N*-(3,5-dichloropyrid-4-yl)benzofurancarboxamide,
2-acetyl-7-methoxy-4-[*N*-(pyrid-4-yl)-N-propyl]benzofurancarboxamide,
2-acetyl-7-methoxy-4-*N*-(2-chloropyrid-3-yl)benzofurancarboxamide,
N-(3,5-dichloropyrid-4-yl)-2-carboxy-7-methoxybenzofuran-4-carboxamide, and
2-ethyl-7-methoxy-4-[*N*-(3-fluoropyridin-4-yl)]benzofurancarboxamide; or a pharmaceutically-acceptable salt thereof.

23. A pharmaceutical composition for therapeutic use, comprising a compound of any of claims 4 to 22 and a pharmaceutically-acceptable carrier or excipient.

24. Use of a compound of any of claims 4 to 22, for the manufacture of a medicament for use in the treatment of a disease state capable of being modulated by inhibition of phosphodiesterase IV or Tumour Necrosis Factor.

25. The use of claim 24, wherein the disease state is a pathological condition associated with a function of phosphodiesterase IV, eosinophil accumulation or a function of the eosinophil.

26. The use of claim 25, wherein the pathological condition is selected from asthma, chronic bronchitis, chronic obstructive airways disease, atopic dermatitis, urticaria, allergic rhinitis, allergic conjunctivitis, vernal conjunctivitis, inflammation of the eye, allergic responses in the eye, eosinophilic granuloma, psoriasis, rheumatoid arthritis, gouty arthritis and other arthritic conditions, ulcerative colitis, Crohn's disease, adult respiratory distress syndrome, diabetes insipidus, keratosis, atopic eczema, atopic dermatitis, cerebral senility, multi-infarct dementia, senile dementia, memory impairment associated with Parkinson's disease, depression, cardiac arrest, stroke and intermittent claudication.

27. The use of claim 25, wherein the pathological condition is selected from chronic bronchitis, allergic rhinitis and adult respiratory distress syndrome.

28. The use of claim 24, wherein the disease state is capable of being modulated by TNF inhibition.

29. The use of claim 28, wherein the disease state is an inflammatory disease or autoimmune disease.

30. The use of claim 29, wherein the disease state is selected from joint inflammation, arthritis, rheumatoid arthritis, rheumatoid spondylitis and osteoarthritis, sepsis, septic shock, endotoxic shock, gram negative sepsis, toxic shock syndrome, acute respiratory distress syndrome, cerebral malaria, chronic pulmonary inflammatory disease, pulmonary sarcoidosis, asthma, bone resorption diseases, reperfusion injury, graft vs host reaction, allograft rejection, malaria, myalgias, HIV, AIDS, ARC, cachexia, Crohn's disease, ulcerative colitis, pyresis, systemic lupus erythematosus, multiple sclerosis, type 1 diabetes mellitus, psoriasis, Bechet's disease, anaphylactoid purpura nephritis, chronic glomerulonephritis, inflammatory bowel disease and leukaemia.

31. The use of claim 25 or claim 28, wherein the pathological condition or disease state is asthma.

32. The use of claim 30, wherein the disease state is acute respiratory distress syndrome, pulmonary inflammatory disease or pulmonary sarcoidosis.

33. The use of claim 30, wherein the disease state is joint inflammation.

34. The use of claim 25 or claim 28, wherein the disease state is a disease or disorder of the brain, such as brain trauma, stroke, ischaemia, Huntingdon's disease or tardive dyskinesia.

35. The use of claim 28, wherein the disease state is a yeast or fungal infection.

36. Use of a compound of any of claims 4 to 22, for the manufacture of a medicament for use in gastroprotection.

37. Use of a compound of any of claims 4 to 22, for the manufacture of a medicament for use as an analgesic, anti-tussive or anti-hyperalgesic in the treatment of neurogenic inflammatory disease associated with irritation and pain.

## Patentansprüche

1. Verwendung einer Verbindung der allgemeinen Formel (i) 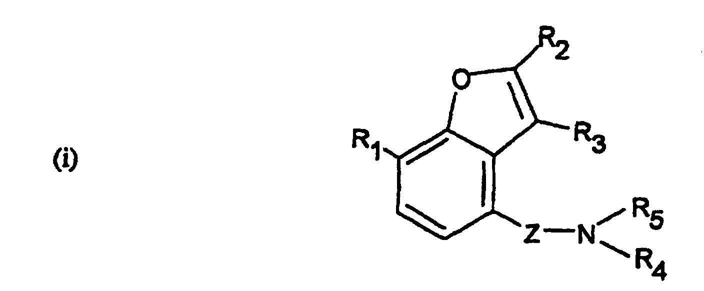 worin Z CO oder CS bedeutet;
R₁ Alkoxy, das gegebenenfalls ein- oder mehrfach durch Halogen substituiert ist, OH oder Thioalkyl bedeutet;
R₂ und R₃ gleich oder verschieden sind und jeweils H, R₆, OR₁₀, COR₆, C(=NOR₆)R₆, Alkyl-C(=NOR₆)R₆, Alkyl-C(=NOH)R₆, C(=NOH)R₆, Halogen, NR₈R₉, CF₃, CN, CO₂H, CO₂R₁₀, CONH₂, CONHR₆ oder CON(R₆)₂ bedeuten;
R₄ H, Arylalkyl, Heteroarylalkyl, Heterocycloalkyl, S(O)ₘR₁₀ oder Alkyl bedeutet, das gegebenenfalls ein- oder mehrfach durch Substituenten substituiert ist, die unter Hydroxy, Alkoxy, CO₂R₇, SO₂NR₁₁R₁₂, CONR₁₁R₁₂, CN, Carbonylsauerstoff, NR₈R₉, COR₁₀ und S(O)ₙR₁₀ ausgewählt sind;
R₅ Aryl, Heteroaryl, Heterocyclo, Arylalkyl, Heteroarylalkyl oder Heterocycloalkyl bedeutet;
wobei in R₄ und/oder R₅ der Aryl/Heteroaryl/Heterocyclo-Teil gegebenenfalls mit einem oder mehreren Substituenten Alkyl-R₁₃ oder R₁₃ substituiert ist;
R₆ die Bedeutung R₁₀ hat, das gegebenenfalls an einer beliebigen Position durch R₁₄ substituiert ist;
R₇ H, Alkyl, Arylalkyl, Heteroarylalkyl oder Heterocycloalkyl bedeutet;
R₈ H, Aryl, Heteroaryl, Heterocyclo, Alkyl, Cycloalkyl, Arylalkyl, Heteroarylalkyl, Heterocycloalkyl, Alkylcarbonyl, Alkoxycarbonyl, Arylsulfonyl, Heteroarylsulfonyl, Heterocyclosulfonyl, Arylcarbonyl, Heteroarylcarbonyl, Heterocyclocarbonyl oder Alkylsulfonyl bedeutet;
R₁₀ Alkyl, Cycloalkyl, Aryl, Heteroaryl, Heterocyclo, Arylalkyl, Heteroarylalkyl oder Heterocycloalkyl bedeutet;
R₉, R₁₁ und R₁₂ gleich oder verschieden sind und jeweils H oder R₁₀ bedeuten;
R₁₃ Alkyl, das gegebenenfalls durch Halogen substituiert ist, Alkoxy, das gegebenenfalls durch Halogen substituiert ist, Aryl, Heteroaryl, Heterocyclo, Hydroxy, Aryloxy, Heteroaryloxy, Heterocyclooxy, Arylalkyloxy, Heteroarylalkyloxy, Heterocycloalkyloxy, CO₂R₇, CONR₁₁R₁₂, SO₂NR₁₁R₁₂, Halogen, -CN, -NR₈R₉, COR₁₀, S(O)ₙR₁₀ oder Carbonylsauerstoff bedeutet;
R₁₄ OH, Carbonylsauerstoff, OR₁₀, NR₈R₉, CN, CO₂H, CO₂R₁₀, CON₁₁R₁₂ oder COR₁₀ bedeutet;
m eine ganze Zahl mit einem Wert bis zu 2 ist; und
n 0-2 bedeutet;
oder eines pharmazeutisch verträglichen Salzes davon;
zur Herstellung eines Arzneimittels zur Behandlung eines Krankheitszustands, der aus folgender Gruppe ausgewählt ist: chronische Bronchitis, chronische obstruktive Atemwegserkrankung, atopische Dermatitis, Nesselsucht, allergische Konjunktivitis, Frühjahrskatarrh, Augenentzündung, allergische Augenreaktionen, eosinophiles Granulom, gichtartige Arthritis, ulzerative Kolitis, Atemnotsyndrom bei Erwachsenen, Diabetes insipidus, Keratose, atopisches Ekzem, zerebrale Senilität, Multiinfarkt-Demenz, senile Demenz, Gedächtnisstörungen bei Parkinson-Krankheit, Depressionen, Herzstillstand, Schlaganfall, intermittierendes Hinken, Gelenkentzündungen, Osteoarthritis, Sepsis, septischer Schock, endotoxischer Schock, gram-negative Sepsis, toxisches Schocksyndrom, akutes Atemnotsyndrom, zerebrale Malaria, chronische Lungenentzündung, pulmonale Sarkoidose, Knochenresorptionskrankheiten, Reperfusionsschädigungen, Graft-versus-Host-Reaktion, Fremdtransplantatabstoßungen, Malaria, Myalgien, ARC, Kachexie, Pyrese, Bechet-Krankheit, anaphylaxieähnliche Purpura-Nephritis, chronische Glomerulonephritis, entzündliche Darmerkrankungen, Leukämie, Hefe- oder Pilzinfektionen, Gehimtrauma, Schlaganfall, Ischämie, Huntington-Krankheit oder tardive Dyskinesie.

2. Verwendung einer Verbindung der Formel (i) gemäß der Definition in Anspruch 1 zur Herstellung eines Arzneimittels zur Verwendung zum Schutz des Magens.

3. Verwendung einer Verbindung der Formel (i) gemäß der Definition in Anspruch 1 zur Herstellung eines Arzneimittels zur Verwendung als Analgetikum, Antitussivum oder Antihyperalgetikum bei der Behandlung einer neurogenen entzündlichen Erkrankung in Verbindung mit Reizungen und Schmerzen.

4. Verbindung der Formel (i) gemäß der Definition in Anspruch 1, mit der Maßgabe, dass die folgenden Verbindungen ausgenommen sind, d. h. Verbindungen, bei denen
R₁ gegebenenfalls substituiertes Alkoxy bedeutet;
R₂ und R₃ gleich oder verschieden sind und jeweils H, Alkyl, Cycloalkyl, Phenyl, Naphtyl oder Heteroaryl (worin Phenyl, Naphtyl oder Heteroaryl gegebenenfalls durch 1 bis 3 Substituenten, die unter OH, Alkoxy, Alkanoyl, Alkoxycarbonyl, NH₂ und CN ausgewählt sind, substituiert sind), Alkanoyl, Cycloalkanoyl, Alkoxycarbonyl, CN, -(CH₂)₁₋₄-COOR₁₀, wenn R₁₀ H, Alkyl, Cycloalkyl, Phenyl, Naphthyl, Heterocyclo oder Arylalkyl bedeutet, oder -(CH₂)₁₋₄-CONR₁₁R₁₂, wenn R₁₁ und R₁₂ jeweils H, Alkyl, Cyctoalkyl, Phenyl, Naphthyl, Heteroaryl oder Arylalkyl darstellen, bedeutet;
R₄ H, Alkyl oder Cycloalkyl bedeutet; und
R₅ 4-Pyridyl bedeutet, das gegebenenfalls in den Positionen 3 und/oder 5 durch Alkyl, OH, Alkoxy, COOH, Alkanoyl, Alkoxy-CO, CF₃, NH₂, CN, NO₂ oder Halogen substituiert ist.

5. Verbindung nach Anspruch 4, wobei R₂ und R₃ gleich oder verschieden sind und jeweils H, R₆, COR₆, C(=NOR₆)R₆, CN, CO₂H, CO₂R₁₀, CONH₂, CONHR₆ oder CON(R₆)₂ bedeuten.

6. Verbindung nach Anspruch 4, wobei
Z die Bedeutung CO hat;
R₁ Alkoxy bedeutet, das gegebenenfalls durch ein oder mehr Halogenatome substituiert ist;
R₂ und R₃ gleich oder verschieden sind und jeweils R₆ₐ oder Alkyl-R₆ₐ bedeuten;
R₆ₐ H, Aryl, Heteroaryl, Heterocyclo, Hydroxy, Alkoxy, Aryloxy, Heteroaryloxy, Heterocyclooxy, Arylalkyloxy, Heteroarylalkyloxy, Heterocycloalkyloxy, Alkylamino, CF₃ oder COR₁₀ bedeutet;
R₁₀ Alkyl, Aryl, Heteroaryl, Heterocyclo, Arylalkyl, Heteroarylalkyl oder Heterocycloalkyl bedeutet;
R₁₃ Aryl, Heteroaryl, Heterocyclo, Hydroxy, Alkoxy, Aryloxy, Heteroaryloxy, Heterocyclooxy, Arylalkoxy, Heteroarylalkyloxy, Heterocycloalkyloxy, CO₂R₇, CONR₁₁R₁₂, SO₂NR₁₁R₁₂, Halogen, CN, NR₈R₉, COR₁₀, S(O)ₙR₁₀ oder Carbonylsauerstoff bedeutet; und
m den Wert 1 oder 2 hat.

7. Verbindung nach Anspruch 4, wobei
R₂ und R₃ jeweils unabhängig voneinander unter H, R₆ und COR₆ ausgewählt sind; und
R₆ Alkyl, Aryl, Heteroaryl, Heterocyclo, Arylalkyl, Heteroarylalkyl oder Heterocycloalkyl bedeutet.

8. Verbindung nach einem der Ansprüche 4 bis 7, wobei R₂ nicht die Bedeutung H hat.

9. Verbindung nach einem der Ansprüche 4 bis 8, wobei R₅ gegebenenfalls substituiertes Aryl oder Heteroaryl bedeutet.

10. Verbindung nach einem der Ansprüche 4 bis 9, wobei R₁ Alkoxy bedeutet, das gegebenenfalls durch ein oder mehr Halogenatome substituiert ist.

11. Verbindung nach einem der Ansprüche 4 bis 10, wobei R₂ und R₃ nicht beide H bedeuten.

12. Verbindung nach einem der Ansprüche 4 bis 11, wobei R₂ und R₃ unabhängig voneinander OR₁₀, Halogen, NR₈R₉ oder CF₃ bedeuten.

13. Verbindung nach einem der Ansprüche 4 bis 11, wobei R₂ und R₃ unabhängig voneinander C(=NOR₆)R₆, Alkyl-C-(=NOR₆)R₆, C(=NOH)R₆ oder Alkyl-C(=NOH)R₆ bedeuten.

14. Verbindung nach einem der Ansprüche 4 bis 11, wobei R₂ und R₃ unabhängig voneinander Heterocydo, Heterocycloalkyl oder Heteroarylalkyl, die gegebenenfalls an einer beliebigen Position (ein- oder mehrfach) durch R₁₄ substituiert sind, bedeuten.

15. Verbindung nach einem der Ansprüche 4 bis 11, wobei R₂ und R₃ unabhängig voneinander Alkyl, das an einer beliebigen Substitution (ein- oder mehrfach) durch OH, OR₁₀, NR₈R₉ oder CN substituiert ist, bedeuten.

16. Verbindung nach einem der Ansprüche 4 bis 11, wobei R₂ und R₃ unabhängig voneinander Alkyl, das ein- oder mehrfach durch COR substituiert ist, bedeuten und R Aryl, Heteroaryl, Heterocyclo (nicht über Stickstoff gebunden), Arylalkyl, Heteroarylalkyl oder Heterocycloalkyl bedeutet.

17. Verbindung nach einem der Ansprüche 4 bis 11, wobei R₂ und R₃ unabhängig voneinander COR gemäß der Definition in Anspruch 16, das gegebenenfalls an einer beliebigen Position (ein- oder mehrfach) durch R₁₄ substituiert ist, bedeuten.

18. Verbindung nach einem der Ansprüche 4 bis 17, wobei R₄ H oder Alkyl bedeutet.

19. Verbindung nach einem der Ansprüche 4 bis 18, wobei R₅ Aryl oder Heteroaryl bedeutet, die jeweils gegebenenfalls ein- oder mehrfach durch die Substituenten Alkyl-R₁₃ oder R₁₃ substituiert sein können.

20. Verbindung nach Anspruch 4, ausgewählt unter
2-Acetyl-7-methoxy-4-N-(2-chlorphenyl)-benzofurancarboxamid,
2-Acetyl-7-methoxy-4-N-(2,6-dimethylphenyl)-benzofurancarboxamid,
2-Acetyl-7-methoxy-4-N-(1-methoxyphenyl)-benzofurancarboxamid,
2-Acetyl-7-methoxy-4-N-(3-brom-5-methylpyrid-2-yl)-benzofurancarboxamid,
2-Acetyl-7-methoxy-4-N-(3-methylphenyl)-benzofurancarboxamid,
2-Acetyl-7-methoxy-4-N-(3,5-dichlorpyrid-2-yl)-benzofurancarboxamid,
2-Acetyl-7-methoxy-4-N-(2-methylphenyl)-benzofurancarboxamid,
2-Acetyl-7-methoxy-4-N-(4-methoxy-2-methylphenyl)-benzofurancarboxamid.
2-Acetyl-7-methoxy-4-N-(pyrimidin-4-yl)-benzofurancarboxamid,
2-Acetyl-7-methoxy-4-N-(2-trifluormethylphenyl)-benzofurancarboxamid,
2-Acetyl-7-methoxy-4-N-[2-(piperidin-1-yl)-phenyl]-benzofurancarboxamid,
2-Acetyl-7-methoxy-4-N-(3-chlorpyrid-4-yl)-benzofurancarboxamid,
2-Acetyl-7-methoxy-4-N-(2-trifluormethoxyphenyl)-benzofurancarboxamid,
2-Acetyl-7-methoxy-4-N-(2-ethylphenyl)-benzofurancarboxamid,
2-Acetyl-7-methoxy-4-N-(2-biphenyl)-benzofurancarboxamid,
2-Acetyl-7-methoxy-4-N-(3-methylpyrid-2-yl)-benzofurancarboxamid,
2-Ethyl-7-methoxy-4-N-(2-chlorpyrid-3-yl)-benzofurancarboxamid,
2-Acetyl-7-methoxy-4-N-(2-methoxyphenyl)-benzofurancarboxamid,
2-Acetyl-7-methoxy-4-N-(2-chlor-6-methylphenyl)-benzofurancarboxamid,
2-(1-Hydroxyethyl)-7-methoxy-4-N-(3,5-dichlorpyrid-4-yl)-benzofurancarboxamid,
2-(3-Pyrid-3-yl-1-oxopropyl)-7-methoxy-4-N-(3,5-dichlorpyrid-4-yl)-benzofurancarboxamid,
2-(1-Benzyloxyimino)-ethyl-7-methoxy-4-N-(3,5-dichlorpyrid-4-yl)-benzofurancarboxamid,
2-Ethyl-7-methoxy-4-N-(3-carboxyphenyl)-benzofurancarboxamid,
2-Ethyl-7-methoxy-4-N-(4-carboxyphenyl)-benzofurancarboxamid und
2-(2,2-Dimethylpropyl)-7-methoxy-4-N-(3,5-dichlorpyrid-4-yl)-benzofurancarboxamid.

21. Verbindung, ausgewählt unter
2-Acetyl-7-methoxy-4-N-(3,5-dichlorpyrid-4-yl)-benzofurancarboxamid und
2-Acetyl-7-methoxy-4-N-(pyrid-4-yl)-benzofurancarboxamid;
oder ein pharmazeutisch verträgliches Salz davon.

22. Verbindung, ausgewählt unter
2-Ethyl-7-methoxy-4-N-(3,5-dichlorpyrid-4-yl)-benzofurancarboxamid,
2-Acetyl-7-methoxy-4-[N-(pyrid-4-yl)-N-propyl]-benzofurancarboxamid,
2-Acetyl-7-methoxy-4-N-(2-chlorpyrid-3-yl)-benzofurancarboxamid,
N-(3,5-dichlorpyrid-4-yl)-2-carboxy-7-methoxybenzofuran-4-carboxamid und
2-Ethyl-7-methoxy-4-[N-(3-fluorpyridin-4-yl)]-benzofurancarboxamid;
oder ein pharmazeutisch verträgliches Salz davon.

23. Pharmazeutische Zusammensetzung zur therapeutischen Verwendung, enthaltend eine Verbindung nach einem der Ansprüche 4 bis 22 und einen pharmazeutisch verträglichen Trägerstoff oder Exzipienten.

24. Verwendung einer Verbindung nach einem der Ansprüche 4 bis 22 zur Herstellung eines Arzneimittels zur Verwendung bei der Behandlung eines Krankheitszustands, der durch die Hemmung von Phosphodiesterase IV oder Tumornekrosefaktor moduliert werden kann.

25. Verwendung nach Anspruch 24, wobei es sich beim Krankheitszustand um einen pathologischen Zustand im Zusammenhang mit einer Funktion von Phosphodiesterase IV, der Eosinophilen-Anreicherung oder einer Funktion der Eosinophilen handelt.

26. Verwendung nach Anspruch 25, wobei der pathologische Zustand aus folgender Gruppe ausgewählt ist: Asthma, chronische Bronchitis, chronische obstruktive Atemwegserkrankung, atopische Dermatitis, Nesselsucht, allergische Rhinitis, allergische Konjunktivitis, Frühjahrskatarrh, Augenentzündung, allergische Augenreaktionen, eosinophiles Granulom, Psoriasis, rheumatoide Arthritis, gichtartige Arthritis und andere arthritische Zustände, ulzerative Kolitis, Morbus Crohn, Atemnotsyndrom bei Erwachsenen, Diabetes insipidus, Keratose, atopisches Ekzem, atopische Dermatitis, zerebrale Senilität, Multiinfarkt-Demenz, senile Demenz, Gedächtnisstörungen bei Parkinson-Krankheit, Depressionen, Herzstillstand, Schlaganfall und intermittierendes Hinken.

27. Verwendung nach Anspruch 25, wobei der pathologische Zustand aus folgender Gruppe ausgewählt ist: chronische Bronchitis, allergische Rhinitis und Atemnotsyndrom bei Erwachsenen.

28. Verwendung nach Anspruch 24, wobei der Krankheitszustand durch TNF-Hemmung moduliert werden kann.

29. Verwendung nach Anspruch 28, wobei es sich beim Krankheitszustand um eine entzündliche Erkrankung oder eine Autoimmunerkrankung handelt.

30. Verwendung nach Anspruch 29, wobei der Krankheitszustand aus folgender Gruppe ausgewählt ist: Gelenkentzündung, Arthritis, rheumatoide Arthritis, rheumatoide Spondylitis und Osteoarthritis, Sepsis, septischer Schock, endotoxischer Schock, gram-negative Sepsis, toxisches Schocksyndrom, akutes Atemnotsyndrom, zerebrale Malaria, chronische Lungenentzündung, pulmonale Sarkoidose, Asthma, Knochenresorptionskrankheiten, Reperfusionsschädigungen, Graft-versus-Host-Reaktion, Fremdtransplantatabstoßungen, Malaria, Myalgien, HIV, AIDS, ARC, Kachexie, Morbus Crohn, ulzerative Kolitis, Pyrese, systemischer Lupus erythematosus, Multiple Sklerose, Typ 1-Diabetes mellitus, Psoriasis, Bechet-Krankheit, anaphylaxieartige Purpura-Nephritis, chronische Glomerulonephritis, entzündliche Darmerkrankungen und Leukämie.

31. Verwendung nach Anspruch 25 oder 28, wobei es sich bei dem pathologischen Zustand oder der Krankheit um Asthma handelt.

32. Verwendung nach Anspruch 30, wobei es sich bei dem Krankheitszustand um akutes Atemnotsyndrom, pulmonale entzündliche Erkrankungen oder pulmonale Sarkoidose handelt.

33. Verwendung nach Anspruch 30, wobei es sich bei dem Krankheitszustand um eine Gelenkentzündung handelt.

34. Verwendung nach Anspruch 25 oder 28, wobei es sich bei dem Krankheitszustand um eine Krankheit oder Störung des Gehirns, z. B. Gehirntrauma, Schlaganfall, Ischämie, Huntington-Krankheit oder tardive Dyskinesie, handelt.

35. Verwendung nach Anspruch 28, wobei es sich bei dem Krankheitszustand um eine Hefe- oder Pilzinfektion handelt.

36. Verwendung einer Verbindung nach einem der Ansprüche 4 bis 22 zur Herstellung eines Arzneimittels zur Verwendung beim Schutz des Magens.

37. Verwendung einer Verbindung nach einem der Ansprüche 4 bis 22 zur Herstellung eines Arzneimittels zur Verwendung als Analgetikum, Antitussivum oder Antihyperalgetikum bei der Behandlung von neurogenen entzündlichen Erkrankungen in Verbindung mit Reizungen und Schmerzen.

## Revendications

1. Utilisation d'un composé répondant à la formule générale (i) 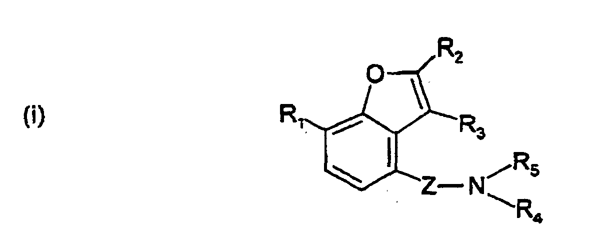 dans laquelle :
Z est CO ou CS ;
R₁ représente un alcoxy éventuellement substitué par un ou plusieurs halogènes, OH ou un thioalkyle ;
R₂ et R₃ sont identiques ou différents et sont, chacun, H, R₆, OR₁₀, COR₆, C(=NOR₆)R₆, alkyl-C(=NOR₆)R₆, alkyl-C(=NOH)R₆, C(=NOH)R₆, un halogène, NR₈R₉, CF₃, CN, CO₂H, CO₂R₁₀, CONH₂, CONHR₆ ou CON(R₆)₂ ;
R₄ représente H, un arylalkyle, un hétéroarylalkyle, un hétérocycloalkyle, S(O)ₘR₁₀ ou un alkyle éventuellement substitué par un ou plusieurs substituants choisis parmi un hydroxy, un alcoxy, CO₂R₇, SO₂NR₁₁R₁₂, CONR₁₁R₁₂, CN, un oxygène de carbonyle, NR₈R₉, COR₁₀ et S(O)ₙR₁₀ ;
R₅ représente un aryle, un hétéroaryle, un hétérocyclo, un arylalkyle, un hétéroarylalkyle ou un hétérocycloalkyle ;
dans R₄ et/ou R₅, la partie aryl/hétéroaryl/hétérocyclo est éventuellement substituée par un ou plusieurs substituants alkyl-R₁₃ ou R₁₃ ;
R₆ représente un R₁₀ éventuellement substitué en une position quelconque par R₁₄ ;
R₇ représente H, un alkyle, un arylalkyle, un hétéroarylalkyle ou un hétérocycloalkyle ;
R₈ représente H, un aryle, un hétéroaryle, un hétérocyclo, un alkyle, un cycloalkyle, un arylalkyle, un hétéroarylalkyle, un hétérocycloalkyle, un alkylcarbonyle, un alcoxycarbonyle, un arylsulfonyle, un hétéroarylsulfonyle, un hétérocyclosulfonyle, un arylcarbonyle, un hétéroarylcarbonyle, un hétérocyclocarbonyle ou un alkylsulfonyle ;
R₁₀ représente un alkyle, un cycloalkyle, un aryle, un hétéroaryle, un hétérocyclo, un arylalkyle, un hétéroarylalkyle ou un hétérocycloalkyle ;
R₉, R₁₁ et R₁₂ sont identiques ou différents et sont, chacun, H ou R₁₀ ;
R₁₃ représente un alkyle éventuellement substitué par un halogène, un alcoxy éventuellement substitué par un halogène, un aryle, un hétéroaryle, un hétérocyclo, un hydroxy, un aryloxy, un hétéroaryloxy, un hétérocyclooxy, un arylalkyloxy, un hétéroarylalkyloxy, un hétérocycloalkyloxy, CO₂R₇, CONR₁₁R₁₂, SO₂NR₁₁R₁₂, un halogène, -CN, -NR₈R₉, COR₁₀, S(O)ₙR₁₀ ou un oxygène de carbonyle ;
R₁₄ représente OH, un oxygène de carbonyle, OR₁₀, NR₈R₉, CN, CO₂H, CO₂R₁₀, CON₁₁R₁₂ ou COR₁₀ ;
m est un nombre entier jusqu'à 2 ; et
n représente un nombre de 0 à 2 ;
ou sel pharmaceutiquement acceptable de celui-ci ;
pour la fabrication d'un médicament destiné au traitement d'un état de maladie choisi parmi la bronchite chronique, la maladie d'obstruction chronique des voies aériennes, la dermatite atopique, l'urticaire, la conjonctivite allergique, la conjonctivite printanière, l'inflammation de l'oeil, les réactions allergiques dans l'oeil, le granulome éosinophilique, l'arthrite goutteuse, la colite ulcéreuse, le syndrome de détresse respiratoire de l'adulte, le diabète insipide, la kératose, l'eczéma atopique, la sénilité cérébrale, la démence à infarctus multiples, la démence sénile, le trouble de la mémoire associé à la maladie de Parkinson, la dépression, l'arrêt cardiaque, l'accident vasculaire cérébral, la claudication intermittente, l'inflammation des articulations, l'arthrose, la septicémie, le choc septique, le choc endotoxique, la septicémie à germes Gram négatif, le syndrome de choc toxique staphilococcique, le syndrome de détresse respiratoire aiguë, la forme cérébrale de la fièvre pernicieuse, la maladie inflammatoire pulmonaire chronique, la sarcoïdose pulmonaire, les maladies de résorption osseuse, la lésion de reperfusion, la réaction du greffon contre l'hôte, le rejet d'allogreffe, la malaria, les myalgies, les syndromes apparentés au SIDA (ARC), la cachexie, la pyresis, la maladie de Bechet, la néphrite à purpura rhumatoïde, la glomérulonéphrite chronique, une affection abdominale inflammatoire, la leucémie, une infection provoquée par des levures ou par des champignons, le trauma cérébral, l'accident vasculaire cérébral, l'ischémie, la chorée de Huntingdon ou la dyskinésie tardive.

2. Utilisation d'un composé de formule (i) comme défini dans la revendication 1, pour la fabrication d'un médicament à utiliser dans une gastroprotection.

3. Utilisation d'un composé de formule (i) comme défini dans la revendication 1, pour la fabrication d'un médicament à utiliser comme analgésique, comme antitussif ou comme médicament contre l'hyperalgésie dans le traitement d'une maladie inflammatoire d'origine nerveuse associée à une irritation et à de la douleur.

4. Composé de formule (i) comme défini dans la revendication 1, à condition que les composés suivants soient exclus, c'est-à-dire ceux dans lesquels :
R₁ est un alcoxy éventuellement substitué ;
R₂ et R₃ sont identiques ou différents et sont, chacun, H, un alkyle, un cycloalkyle, un phényle, un naphtyle ou un hétéroaryle (où le phényle, le naphtyle ou l'hétéroaryle est éventuellement substitué par de 1 à 3 substituants choisis-parmi OH, un alcoxy, un alcanoyle, un alcoxycarbonyle, NH₂ et CN), un alcanoyle, un cycloalcanoyle, un alcoxycarbonyle, CN, -(CH₂)₁₋₄-COOR₁₀ lorsque R₁₀ est H, un alkyle, un cycloalkyle, un phényle, un naphtyle, un hétérocyclo ou un arylalkyle, ou -(CH₂)₁₋₄-CONR₁₁R₁₂ lorsque R₁₁ et R₁₂ sont, chacun, H, un alkyle, un cycloalkyle, un phényle, un naphtyle, un hétéroaryle ou un arylalkyle ;
R₄ est H, un alkyle ou un cycloalkyle ; et
R₅ est un 4-pyridyle éventuellement substitué en les positions 3 et/ou 5 par un alkyle, OH, un alcoxy, COOH, un alcanoyle, un alcoxy-CO-, CF₃, NH₂, CN, NO₂ ou un halogène.

5. Composé selon la revendication 4, dans lequel R₂ et R₃ sont identiques ou différents et sont, chacun, H, R₆, COR₆, C(=NOR₆)R₆, CN, CO₂H, CO₂R₁₀, CONH₂, CONHR₆ ou CON(R₆)₂.

6. Composé selon la revendication 4, dans lequel :
Z est CO ;
R₁ est un alcoxy éventuellement substitué par un ou plusieurs halogènes ;
R₂ et R₃ sont identiques ou différents et sont, chacun, R₆ₐ ou un alkyl-R₆ₐ ;
R₆ₐ est H, un aryle, un hétéroaryle, un hétérocyclo, un hydroxy, un alcoxy, un aryloxy, un hétéroaryloxy, un hétérocyclooxy, un arylalkyloxy, un hétéroarylalkyloxy, un hétérocycloalkyloxy, un alkylamino, CF₃ ou COR₁₀ ;
R₁₀ est un alkyle, un aryle, un hétéroaryle, un hétérocyclo, un arylalkyle, un hétéroarylalkyle ou un hétérocycloalkyle ;
R₁₃ est un aryle, un hétéroaryle, un hétérocyclo, un hydroxy, un alcoxy, un aryloxy, un hétéroaryloxy, un hétérocyclooxy, un arylalcoxy, un hétéroarylalkyloxy, un hétérocycloalkyloxy, CO₂R₇, CONR₁₁R₁₂, SO₂NR₁₁R₁₂, un halogène, CN, NR₈R₉, COR₁₀, S(O)ₙR₁₀ ou un oxygène de carbonyle ; et
m est égal à 1 ou à 2.

7. Composé selon la revendication 4, dans lequel
R₂ et R₃ sont, chacun indépendamment, choisis parmi H, R₆ et COR₆ ; et
R₆ est un alkyle, un aryle, un hétéroaryle, un hétérocyclo, un arylalkyle, un hétéroarylalkyle ou un hétérocycloalkyle.

8. Composé selon l'une quelconque des revendications 4 à 7, dans lequel R₂ n'est pas H.

9. Composé selon l'une quelconque des revendications 4 à 8, dans lequel R₅ est un hétéroaryle ou aryle éventuellement substitué.

10. Composé selon l'une quelconque des revendications 4 à 9, dans lequel R₁ est un alcoxy éventuellement substitué par un ou plusieurs halogènes.

11. Composé selon l'une quelconque des revendications 4 à 10, dans lequel R₂ et R₃ ne sont pas tous deux H.

12. Composé selon l'une quelconque des revendications 4 à 11, dans lequel R₂ et R₃ sont, indépendamment, OR₁₀, un halogène, NR₈R₉ ou CF₃.

13. Composé selon l'une quelconque des revendications 4 à 11, dans lequel R₂ et R₃ sont, indépendamment, C(=NOR₆)R₆, alkyl-C(=NOR₆)R₆, C(=NOH)R₆ ou alkyl-C(=NOH)R₆.

14. Composé selon l'une quelconque des revendications 4 à 11, dans lequel R₂ et R₃ sont, indépendamment, un hétérocyclo, un hétérocycloalkyle ou un hétéroarylalkyle éventuellement substitué en une position quelconque par (un ou plusieurs) R₁₄.

15. Composé selon l'une quelconque des revendications 4 à 11, dans lequel R₂ et R₃ sont, indépendamment, un alkyle substitué en une position quelconque par (un ou plusieurs) OH, OR₁₀, NR₈R₉ ou CN.

16. Composé selon l'une quelconque des revendications 4 à 11, dans lequel R₂ et R₃ sont, indépendamment, un alkyle substitué par un ou plusieurs COR et R est un aryle, un hétéroaryle, un hétérocyclo (non lié par un azote), un arylalkyle, un hétéroarylalkyle ou un hétérocycloalkyle.

17. Composé selon l'une quelconque des revendications 4 à 11, dans lequel R₂ et R₃ sont, indépendamment, COR comme défini dans la revendication 16 éventuellement substitué en une position quelconque par (un ou plusieurs) R₁₄.

18. Composé selon l'une quelconque des revendications 4 à 17, dans lequel R₄ est H ou un alkyle.

19. Composé selon l'une quelconque des revendications 4 à 18, dans lequel R₅ est un aryle ou un hétéroaryle, l'un ou l'autre pouvant être éventuellement substitué par un ou plusieurs substituants alkyl-R₁₃ ou R₁₃.

20. Composé selon la revendication 4, choisi parmi :
le 2-acétyl-7-méthoxy-4-*N*-(2-chlorophényl)benzofurancarboxamide,
le 2-acétyl-7-méthoxy-4-*N*-(2,6-diméthylphényl)benzofurancarboxamide,
le 2-acétyl-7-méthoxy-4-*N*-(4-méthoxyphényl)benzofurancarboxamide,
le 2-acétyl-7-méthoxy-4-*N*-(3-bromo-5-méthylpyrid-2-yl)-benzofurancarboxamide,
le 2-acétyl-7-méthoxy-4-*N*-(3-méthylphényl)benzofurancarboxamide,
le 2-acétyl-7-méthoxy-4-*N*-(3,5-dichloropyrid-2-yl)-benzofurancarboxamide,
le 2-acétyl-7-méthoxy-4*-N*-(2-méthylphényl)benzofurancarboxamide,
le 2-acétyl-7-méthoxy-4-*N*-(4-méthoxy-2-méthylphényl)-benzofurancarboxamide,
le 2-acétyl-7-méthoxy-4-*N*-(pyrimidin-4-yl)benzofurancarboxamide,
le 2-acétyl-7-méthoxy-4-*N*-(2-trifluorométhylphényl)-benzofurancarboxamide,
le 2-acétyl-7-méthoxy-4-*N*-[2-(pipéridin-1-yl)phényl]-benzofurancarboxamide,
le 2-acétyl-7-méthoxy-4-*N*-(3-chloropyrid-4-yl) benzofurancarboxamide,
le 2-acétyl-7-méthoxy-4-*N*-(2-trifluorométhoxyphényl) - benzofurancarboxamide,
le 2-acétyl-7-méthoxy-4-*N*-(2-éthylphényl)benzofurancarboxamide,
le 2-acétyl-7-méthoxy-4*-N-*(2-biphényl)benzofurancarboxamide,
le 2-acétyl-7-méthoxy-4*-N-*(3-méthylpyrid-2-yl)benzofurancarboxamide,
le 2-éthyl-7-méthoxy-4*-N-*(2-chloropyrid-3-yl)benzofurancarboxamide,
le 2-acétyl-7-méthoxy-4-*N*-(2-méthoxyphényl)benzofurancarboxamide,
le 2-acétyl-7-méthoxy-4*-N-*(2-chloro-6-méthylphényl)-benzofurancarboxamide,
le 2-(1-hydroxyéthyl)-7-méthoxy-4-*N*-(3, 5-dichloropyrid-4-yl)-benzofurancarboxamide,
le 2-(3-pyrid-3-yl-1-oxopropyl)-7-méthoxy-4-*N*-(3, 5-dichloropyrid-4-yl)benzofurancarboxamide,
le 2-(1-benzyloxyimino)éthyl-7-méthoxy-4*-N-*(3,5-dichloropyrid-4-yl)benzofurancarboxamide,
le 2-éthyl-7-méthoxy-4*-N*-(3-carboxyphényl)benzofurancarboxamide,
le 2-éthyl-7-méthoxy-4-*N*-(4-carboxyphényl)benzofurancarboxamide, et
le 2-(2, 2-diméthylpropyl)-7-méthoxy-4-*N*-(3,5-dichloropyrid-4-yl)benzofurancarboxamide.

21. Composé choisi parmi :
le 2-acétyl-7-méthoxy-4-*N*-(3,5-dichloropyrid-4-yl)-benzofurancarboxamide et
le 2-acétyl-7-méthoxy-4-*N*-(pyrid-4-yl)benzofurancarboxamide ;
ou sel pharmaceutiquement acceptable de celui-ci.

22. Composé choisi parmi :
le 2-éthyl-7-méthoxy-4-*N*-(3,5-dichloropyrid-4-yl)-benzofurancarboxamide,
le 2-acétyl-7-méthoxy-4-[*N*-(pyrid-4-yl)-N-propyl]-benzofurancarboxamide,
le 2-acétyl-7-méthoxy-4-*N*-(2-chloropyrid-3-yl)-benzofurancarboxamide,
le N-(3,5-dichloropyrid-4-yl)-2-carboxy-7-méthoxybenzofuran-4-carboxamide, et
le 2-éthyl-7-méthoxy-4-[*N*-(3-fluoropyridin-4-yl)]-benzofurancarboxamide ;
ou sel pharmaceutiquement acceptable de celui-ci.

23. Composition pharmaceutique destinée à une utilisation thérapeutique, comprenant un composé selon l'une quelconque des revendications 4 à 22 et un véhicule ou excipient pharmaceutiquement acceptable.

24. Utilisation d'un composé selon l'une quelconque des revendications 4 à 22, pour la fabrication d'un médicament à utiliser dans le traitement d'un état de maladie pouvant être modulé par l'inhibition de la phosphodiestérase de type IV ou du facteur nécrosant des tumeurs.

25. Utilisation selon la revendication 24, dans laquelle l'état de maladie est un état pathologique associé à une fonction de la phosphodiestérase de type IV, à une accumulation d'éosinophiles ou à une fonction de l'éosinophile.

26. Utilisation selon la revendication 25, dans laquelle l'état pathologique est choisi parmi l'asthme, la bronchite chronique, la maladie d'obstruction chronique des voies aériennes, la dermatite atopique, l'urticaire, la rhinite allergique, la conjonctivite allergique, la conjonctivite printanière, l'inflammation de l'oeil, les réactions allergiques dans l'oeil, le granulome éosinophilique, le psoriasis, la polyarthrite rhumatoïde, l'arthrite goutteuse et d'autres états arthritiques, la colite ulcéreuse, la maladie de Crohn, le syndrome de détresse respiratoire de l'adulte, le diabète insipide, la kératose, l'eczéma atopique, la dermatite atopique, la sénilité cérébrale, la démence à infarctus multiples, la démence sénile, le trouble de la mémoire associé à la maladie de Parkinson, la dépression, l'arrêt cardiaque, l'accident vasculaire cérébral et la claudication intermittente.

27. Utilisation selon la revendication 25, dans laquelle l'état pathologique est choisi parmi la bronchite chronique, la rhinite allergique et le syndrome de détresse respiratoire de l'adulte.

28. Utilisation selon la revendication 24, dans laquelle l'état de maladie est capable d'être modulé par une inhibition du TNF.

29. Utilisation selon la revendication 28, dans laquelle l'état de maladie est une maladie inflammatoire ou une maladie auto-immune.

30. Utilisation selon la revendication 29, dans laquelle l'état de maladie est choisi parmi l'inflammation des articulations, l'arthrite, la polyarthrite rhumatoïde, la pelvispondylite rhumatismale et l'arthrose, la septicémie, le choc septique, le choc endotoxique, la septicémie à germes Gram négatif, le syndrome de choc toxique staphilococcique, le syndrome de détresse respiratoire aiguë, la forme cérébrale de la fièvre pernicieuse, la maladie inflammatoire pulmonaire chronique, la sarcoïdose pulmonaire, l'asthme, les maladies de résorption osseuse, la lésion de reperfusion, la réaction du greffon contre l'hôte, le rejet d'allogreffe, la malaria, les myalgies, le VIH, le SIDA, les syndromes apparentés au SIDA (ARC), la cachexie, la maladie de Crohn, la colite ulcéreuse, la pyresis, le lupus érythémateux aigu disséminé, la sclérose en plaques, le diabète sucré de type 1, le psoriasis, la maladie de Bechet, la néphrite à purpura rhumatoïde, la glomérulonéphrite chronique, une affection abdominale inflammatoire et la leucémie.

31. Utilisation selon la revendication 25 ou 28, dans laquelle l'état pathologique ou l'état de maladie est l'asthme.

32. Utilisation selon la revendication 30, dans laquelle l'état de maladie est le syndrome de détresse respiratoire aiguë, la maladie inflammatoire pulmonaire ou la sarcoïdose pulmonaire.

33. Utilisation selon la revendication 30, dans laquelle l'état de maladie est l'inflammation des articulations.

34. Utilisation selon la revendication 25 ou 28, dans laquelle l'état de maladie est une maladie ou un trouble du cerveau, tel qu'un trauma cérébral, l'accident vasculaire cérébral, l'ischémie, la maladie de Huntingdon ou la dyskinésie tardive.

35. Utilisation selon la revendication 28, dans laquelle l'état de maladie est une infection provoquée par des levures ou par des champignons.

36. Utilisation d'un composé selon l'une quelconque des revendications 4 à 22, pour la fabrication d'un médicament à utiliser dans une gastroprotection.

37. Utilisation d'un composé selon l'une quelconque des revendications 4 à 22, pour la fabrication d'un médicament à utiliser comme analgésique, comme antitussif ou comme médicament contre l'hyperalgésie dans le traitement d'une maladie inflammatoire d'origine nerveuse associée à une irritation et à de la douleur.
